# FASCICULE DE BREVET EUROPEEN

(11) **EP 2 474 553 B1**
(45) Date de publication et mention de la délivrance du brevet: **21.01.2015**
(21) Numéro de dépôt: 12163474.5
(22) Date de dépôt: 21.03.2008
(51) Int. Cl.: C07K 7/06, A61K 49/08, A61K 49/14, A61K 49/18, C07D 257/02, C07D 403/06, B82Y 5/00, G01N 33/68

(54) **Composes pour le diagnostique de l'apoptose**
Verbindungen zur Diagnose der Apoptose
Compounds for diagnosing apoptosis

(30) Priorité: 28.03.2007 FR 0754086
(43) Date de publication de la demande: 11.07.2012
(62) Demande divisionnaire de: 08718146.7
(73) Titulaire: GUERBET, 93420 Villepinte (FR)
(72) Inventeur: Port, Marc, 95170 DEUIL LA BARRE (FR); Rousseaux, Olivier, 60300 SENLIS (FR); Muller, Robert, 7000 MONS (BE); Burtea, Carmen, 7000 MONS (BE)
(74) Mandataire: Regimbeau

(56) Documents cités:
- WO-A-2006/032705
- WO-A-2007/042504
- WO-A1-2007/042506
- WO-A2-03/059397
- WO-A2-2004/112840
- US-A1- 2002 122 806
- US-A1- 2007 048 808
- LAUMONIER C ET AL: "A new peptidic vector for molecular imaging of apoptosis, identified by phage display technology", JOURNAL OF BIOMOLECULAR SCREENING, LARCHMONT, NY, US, vol. 11, no. 5, 1 août 2006 (2006-08-01), pages 537-545, XP008085196, ISSN: 1087-0571
- SCHELLENBERGER E A ET AL: "Magneto/Optical Annexin V, a Multimodal Protein", BIOCONJUGATE CHEMISTRY, ACS, WASHINGTON, DC, US, vol. 15, 1 janvier 2004 (2004-01-01), pages 1062-1067, XP002456551, ISSN: 1043-1802
- DATABASE PUBMED 01 Décembre 1999 HARRIS JR AND MARKL J: 'Keyhole limpet hemocyanin (KLH): a biomedical review' Retrieved from Micron. 30(6): 597-623
- SHTATLAND T ET AL: "PepBank-a database of peptides based on sequence text mining and public peptide data sources", BMC BIOINFORMATICS, vol. 8, 1 August 2007 (2007-08-01), DOI: 10.1186/1471-2105-8-280

## Description

L'invention concerne de nouveaux composés de diagnostique de maladies liées à l'apoptose, leur procédé de préparation et leur utilisation en imagerie médicale.

L'apoptose, ou mort cellulaire programmée, fait référence aux mécanismes qui aboutissent au suicide des cellules eucaryotes en réponse à certains stimuli. Ce phénomène physiologique assure l'équilibre entre les cellules qui prolifèrent et celles qui doivent être naturellement éliminées. En situation pathologique, il peut être accru et avoir des conséquences dévastatrices sur le plan fonctionnel dans certains tissus. Le rôle de l'apoptose a été largement démontré dans les maladies neurodégénératives chroniques (maladie d'Alzheimer, maladie de Parkinson), dans les maladies ischémiques (infarctus du myocarde, ischémie cérébrale) et dans les pathologies inflammatoires (athérosclérose, arthrite). Inversement, l'apoptose peut jouer un rôle bénéfique lorsqu'elle conduit à la mort des cellules tumorales lors des traitements par radiothérapie ou par chimiothérapie

La détection de l'apoptose en imagerie constitue un nouvel outil diagnostique très utile pour aider les cliniciens à apprécier la sévérité d'une pathologie, à prédire le risque de survenue d'événement grave et à suivre l'efficacité des traitements grâce à une caractérisation fine des lésions.

La détection de l'apoptose in vivo est difficile en raison notamment du fait que le phénomène n'est pas toujours exprimé, et qu'il est relativement bref puisqu'il se déroule sur une période de 6 à 24 heures maximum, après quoi les débris cellulaires sont éliminés par les cellules phagocytaires.

L'art antérieur décrit plusieurs tentatives de mise au point d'agents de contraste destinés à cibler des cellules apoptotiques Ces agents comprennent typiquement une partie de ciblage appelée biovecteur, destinée à la reconnaissance spécifique de la cible biologique associée au mécanisme d'apoptose, et une partie de signal capable d'être détectée par les méthodes d'imagerie : imagerie optique, scintigraphie, imagerie par résonance magnétique (IRM). La partie de signal est par exemple un chélate de métal paramagnétique tel que le Gadolinium, linéaire ou macrocyclique, notamment DTPA, DTPA BMA, DTPA BOPTA, DO3A, DOTA. Concernant la partie de ciblage, la particularité des cellules apoptotiques à exposer la phosphatidylsérine (PS) sur le feuillet externe de la membrane plasmique (alors qu'elle est normalement confinée sur le feuillet interne) a conduit à chercher des biovecteurs ciblant la PS. L'externalisation de la PS survient précocement et peut donc jouer un rôle de marqueur du processus apoptotique.

Plusieurs protéines possèdent une forte affinité et une grande spécificité pour la PS. C'est en particulier de l'annexine V. Il s'agit d'une protéine endogène de 35 kDa de poids moléculaire, présente au niveau intra ou extra cellulaire. Elle a été couplée à différents groupes rapporteurs selon les modalités d'imagerie retenues. En particulier, elle a été conjuguée à des particules d'oxyde de fer superparamagnétiques (Schellenberger EA. et al., Mol. Imaging, 2002, 1:102-107). In vitro, cet agent de contraste a permis d'obtenir une image en IRM dans des cellules Jurkat rendues apoptotiques après traitement par la camptothécine.

Actuellement, la modalité de détection de l'apoptose in vivo la plus utilisée est la scintigraphie à l'aide d'annexine V couplée au 99mTc. Dans un modèle d'athérosclérose chez le lapin soumis à une désendothélialisation de l'aorte abdominale et à un régime riche en cholestérol, le marqueur s'est accumulé massivement dans les lésions (Kolodgie FD. et al., Circulation, 2003, 108:3134-3139).
US 2007/048808 a trait à une méthode visant à identifier des individus présentant un risque de développer un carcinome hépatocellulaire, ladite méthode détectant des formes d'expression de gènes causés par la présence de l'antigène x du virus de l'hépatite B. US 2007/048808 ne cite pas l'apoptose. US 2007/048808 décrit en particulier la préparation de réactifs de type peptidiques et anticorps pour un criblage (« screening ») de carcinome hépatocellulaire, faisant notamment intervenir un peptide L4B, couplé par l'intermédiaire de l'atome de soufre libre de la cystéine à l'enzyme KLH ou « Keyhole limpet hemocyanin » (voir exemple 3 page 7). Le peptide L4B comprend la séquence SEQ ID N°3 selon la présente invention. Cependant, dans le document US 2007/048808, la KLH n'est pas utilisée en tant que marqueur pour imagerie optique.
Les documents WO 03/059397, US 2002/122806 et WO 2004/112840 décrivent des peptides conjugués à une entité signal et leur utilisation en tant qu'agent de diagnostic de maladies, mais ne divulguent pas un peptide comprenant la séquence SEQ ID NO: 3. Le document WO 2006/032705 mentionne un peptide comprenant la séquence SEQ ID NO: 3 sous forme d'un biovecteur peptidique lipophile.
Les documents WO 2007/042504 et WO 2007/042506 divulguent des composés spécifiques exclus du champ de la présente invention.
L'article du laumonier C et al. (Journal of Biomolecular Screening 2006: 537-545) divulgue des hexapeptides ciblant l'apoptose.

On recherche toujours une amélioration de la qualité du diagnostic in vivo à l'aide de nouveaux marqueurs très spécifiques, destinés aux modalités d'imagerie connues de l'homme du métier, notamment l'IRM, les rayons X, la scintigraphie aux rayons gamma, le scanner CT, les ultrasons, le PET, l'imagerie optique. On rappelle que dans le cas de l'IRM, un contraste est obtenu grâce à l'administration d'agents de contraste contenant des métaux paramagnétiques ou des composés superparamagnétiques qui ont un effet sur la relaxivité des protons de l'eau. Dans le cas de la scintigraphie, le contraste est obtenu par la localisation spécifique d'un composé radiopharmaceutique émettant des rayons gamma ou beta.

On cherche des composés dont la synthèse chimique n'est pas trop complexe, suffisamment stables in vivo pour une utilisation en imagerie médicale, et dont le prix de revient n'est pas trop élevé et donc en particulier des composés ne comprenant pas de biovecteur protéique telle que l'annexine, notamment pour l'IRM.

On cherche aussi pour l'oncologie des produits permettant une imagerie des signes précoces d'apoptose afin notamment d'aider à sélectionner les traitements de chimiothérapie les plus efficaces. L'activité des drogues antihormonales, anti-angiogéniques et la radiothérapie dépendent principalement de l'apoptose des cellules tumorales, c'est pourquoi le suivi en imagerie de ce phénomène apportera des informations critiques pour la prise en charge des patients.

Dans les maladies ischémiques cérébrales ou cardiaques, l'apoptose survient généralement 24 à 48 heures après l'accident initial. Des médicaments anti-apoptotiques, agissant par exemple sur la voie des caspases, seraient certainement utiles s'ils pouvaient être associés à une méthode d'imagerie. Il en est de même pour l'utilisation de drogues immunosuppressives dans les cas de rejet de greffe d'organe. Il existe donc de nombreuses indications pour un agent de contraste capable de détecter efficacement les cellules apoptotiques in vivo.

Le demandeur a réussi à obtenir des composés comprenant une partie de ciblage de zones d'apoptose, efficaces non seulement in vitro mais aussi et surtout en IRM in vivo. De tels composés sont en effet difficiles à obtenir car il faut non seulement identifier un biovecteur efficace in vitro, mais obtenir un composé efficace en imagerie de diagnostique clinique humaine. C'est particulièrement le cas pour l'IRM qui est reconnue pour être une technique très demandée car sans radioactivité, mais dont la sensibilité est très nettement inférieure à celle de la médecine nucléaire.

Le composé obtenu doit à la fois avoir une affinité suffisante pour reconnaître sa cible, une forte spécificité pour être un indicateur distinctifde l'état pathologique, une stabilité appropriée pour ne pas être dégradé ou modifié in vivo ; et en plus sans que la partie de signal n'interfère de manière gênante sur ces différents paramètres (par exemple sans altérer l'affinité et la stabilité du ligand)
Dans la présente demande on utilise le tableau de correspondance suivant :

| | | |
|---|---|---|
| Alanine | A | Ala |
| Arginine | R | Arg |
| Asparagine | N | Asn |
| Aspartate | D | Asp |
| Cystéine | C | Cys |
| Glutamate | E | Glu |
| Glutamine | Q | Gln |
| Glycine | G | Gly |
| Histidine | H | His |
| Isoleuciune | I | Ile |
| Leucine | L | Leu |
| Lysine | K | Lys |
| Méthionine | M | Met |
| Phénylalanine | F | Phe |
| Proline | P | Pro |
| Sérine | S | Ser |
| Thréonine | T | Thr |
| Tryptophane | W | Trp |
| Tyrosine | Y | Tyr |
| Valine | V | Val |

Après de nombreuses tentatives, le demandeur a réussi à obtenir des composés efficaces in vivo.

L'invention concerne ainsi un composé de formule générale (I) suivante :

Signal - Lien - Peptide (I)

dans laquelle
Signal représente une entité signal ;
Lien, présent ou non, représente une liaison chimique et
Peptide représente un peptide comprenant un peptide ciblant l'apoptose, le peptide ciblant l'apoptose étant choisi parmi les peptides de formule suivante
- P-G-D-L-X8-X9 (3) (SEQ ID No 3)
dans laquelle X8 représente la sérine ou la valine et X9 représente la thréonine ou l'arginine ;

Le demandeur a en effet démontré l'affinité des peptides de formule (3) pour la PS comme cela sera décrit plus loin.

Par l'expression peptide ciblant l'apoptose, et maladie associée à l'apoptose, on entend que les composés du demandeur sont des composés capables de cibler des zones biologiques (cellules, tissus, organes ...) subissant un mécanisme d'apoptose, ce mécanisme se traduisant par une maladie liée à l'apoptose à un stade précoce ou déjà avancé. Cette information diagnostique permet alors au clinicien un traitement plus approprié, tel que l'administration d'un médicament anti-apoptose adapté au territoire biologique concerné, et le cas échéant d'éventuels diagnostiques complémentaires. Ainsi l'invention concerne des composés de formule I et leur utilisation pour le ciblage de zones (une ou plusieurs) biologiques (cellules, tissus, organes ...) subissant un mécanisme d'apoptose.

On entend par entité Signal une entité chimique permettant d'obtenir un signal en imagerie médicale, en particulier :
- un chélate capable d'être couplé à un métal paramagnétique ou à un radionucléide,
- une nanoparticule métallique, notamment une nanoparticule superparamagnétique d'oxyde de fer

Dans un mode de réalisation avantageux, Signal représente une entité signal choisie parmi :
- un chélate couplé à un métal paramagnétique M ou à un radionucléide,
- une nanoparticule superparamagnétique revêtue d'une couche organique, de préférence ayant un noyau en oxyde de fer et
- une molécule fluorescente utilisée en imagerie optique ; les composés suivants sont exclus des composés de formule I selon la présente invention :

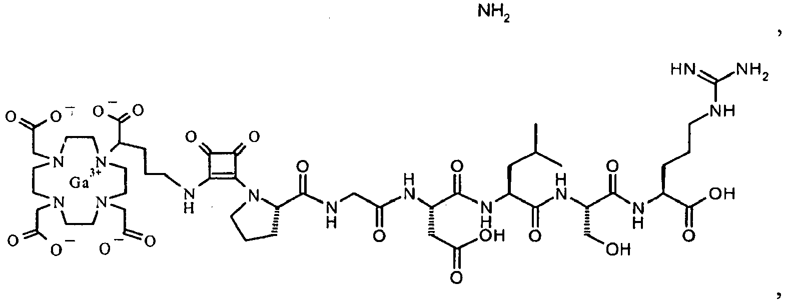

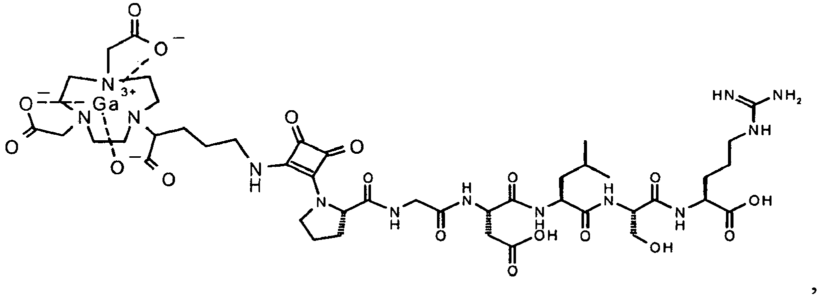

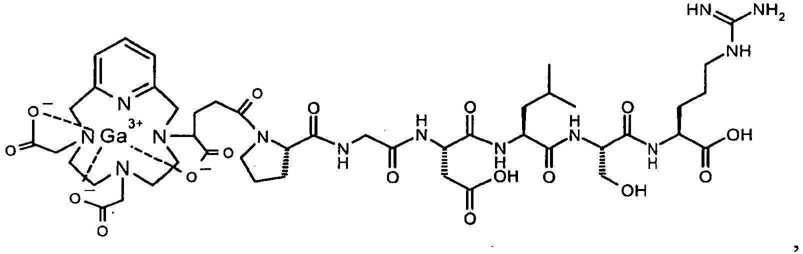

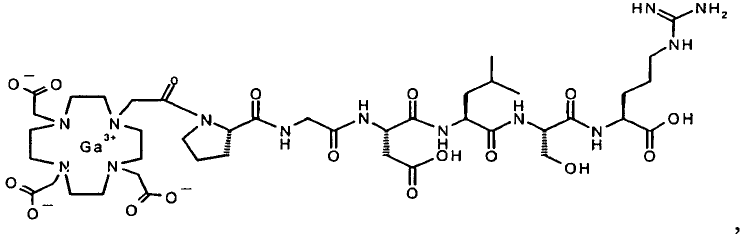

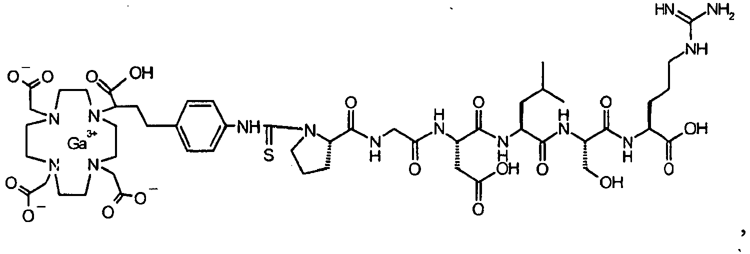

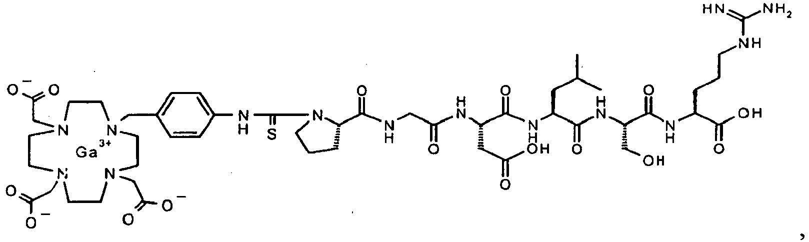

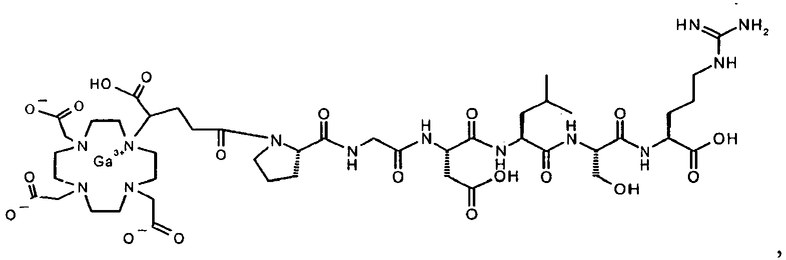

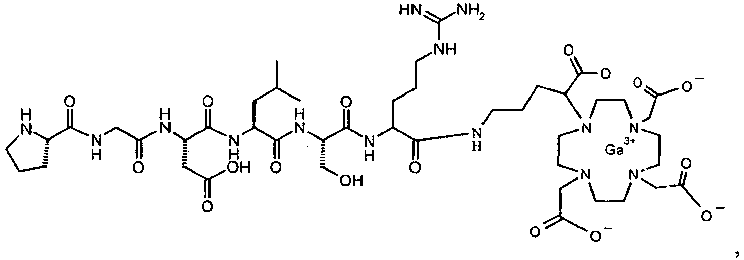

Selon un mode de réalisation, l'entité signal comprend au moins un chélate Ch (sous forme complexant un métal M) couplé au métal M. Avantageusement, le métal M est un ion de métal paramagnétique, ou un radionucléide. De façon avantageuse il s'agit d'un métal paramagnétique. Le complexe formé par le chélate et le métal M est stable dans les conditions physiologiques de manière à éviter une libération non souhaitée du métal M dans l'organisme. Avantageusement le chélate ou l'entité signal comprend au moins un groupe fonctionnel permettant de relier l'entité signal avec le Lien ou directement avec le Peptide.

Avantageusement, Ch est un chélate linéaire choisi parmi : EDTA, DTPA diéthylènetriaminopentaacétique acide, N-[2-[bis(carboxyméthyl)amino]-3-(4-ethoxyphenyl)propyl]-N-[2-[bis(carboxyméthyl)amino]éthyle]-L-glycine (EOB-DTPA), , des dérivés mono- ou bis-amide du DTPA, tels que N,N-bis[2-[carboxyméthyl[(méthylcarbamoyl)méthyle]amino]éthyle] glycine (DTPA-BMA), 4-carboxy-5,8,11-tris(carboxyméthyl)-1-phényl-2-oxa-5,8,11-triazatridecan-13-oique acide (BOPTA).

De façon avantageuse, Ch est un chélate macrocyclique choisi parmi 1,4,7,10-tétraazacyclododécan-1,4,7,10-tétraacétique acide (DOTA), 1,4,7,10-tétraazacyclododécan-1,4,7-triacétique acide (DO3A) 10-(2-hydroxypropyl)-1,4,7,10-tétraazacyclododécan-1,4,7-triacétique acide (HPDO3A) 2-méthyl-1,4,7,10-tétraazacyclododécan-1,4,7,10-tétraacétique acide (MCTA), ( alpha , alpha ', alpha ", alpha "')-tétraméthyl-1,4,7,10-tétraazacyclododécan-1,4,7,10-tétraacétique acide (DOTMA), 3,6,9,15-tétraazabicyclo[9.3.1]pentadéca-1(15),11,13-triène-3,6,9-triacétique acide (PCTA), 1,4,7-triazacyclononane N,N',N44-triacétique acide (NOTA), AAZTA (décrit notamment dans WO 2006/00273 formule III page 120 et US 2006/00118830 page 2 et 89), TETA, TETMA, PDTA, et leurs dérivés benzo, LICAM, MECAM, HOPO (DE 102004062258).

Ch peut également être un dérivé de ces composés dans lesquels un ou plusieurs groupes carboxyliques sont sous forme d'un sel, ester, amide correspondant ; ou un composé correspondant dans lequel un ou plusieurs groupes carboxyliques sont remplacés par un groupe phosphonique et/ou phosphinique.
On peut aussi utiliser un chélate choisi parmi : DOTA gadofluorines, DO3A, HPDO3A, TETA, TRITA, HETA, DOTA-NHS, M4DOTA, M4DO3A, PCTA et leurs dérivés, avantageusement choisi parmi. DOTA, DTPA, DO3A, HPDO3A, TRITA, TETA, BOPTA, NOTA, PCTA, DOTMA, AAZTA, HOPO et leurs dérivés.
De manière plus large, le ou les chélates formant l'entité signal pourront répondre à la formule du document WO01/60416 suivante :

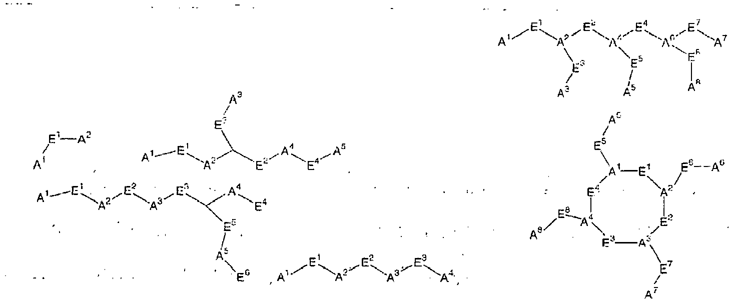

On pourra utiliser en particulier les composés DTPA, DOTA, NOTA, DO3A, et dérivés. On citera aussi les chélates rappelés dans WO03/011115 notamment de formules suivantes : 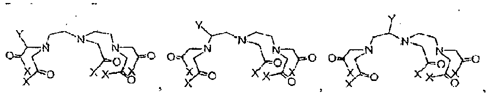 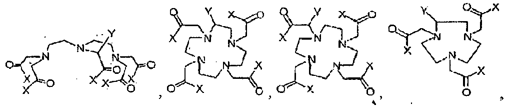 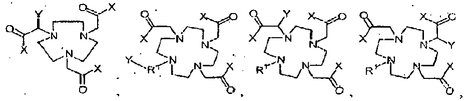 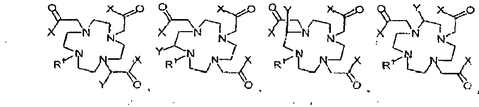 and 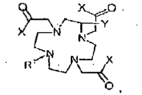 avec X un groupe capable de coordiner un cation métallique, de préférence O-, OH, NH₂, OPO₃-, NH avec R une chaîne aliphatique, Y un lien chimique.

On pourra en particulier utiliser les chélates désignés P730 du demandeur décrits dans EP 661 279 (US 5 919 432) de formule 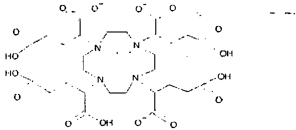 et les chélates à squelette PCTA décrits par le demandeur notamment dans US 6 440 956, que ces chélates ou leurs intermédiaires soient porteurs ou non de chaînes hydrophiles, et en particulier de chaînes aminoalcool courtes ou longues. 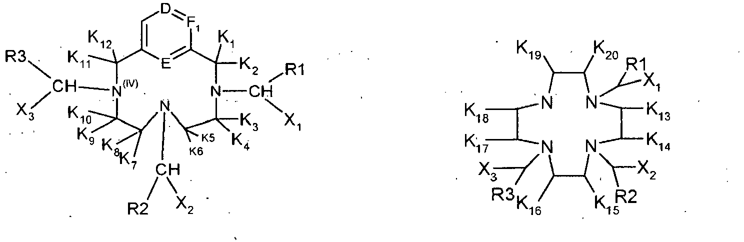 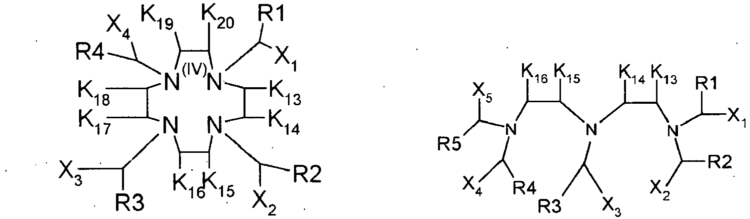 avec X1 à X4 et K1 à K16 des chélates ci-dessus représentant H ou une chaîne en C₁-C₂₀, et R1, R2, R3, R4, R5 représentant indépendamment -COOH, -P(O)(OH)₂ et étant choisis de façon à ce que le chélate comprenne au moins une fonction capable d'être couplée à un PEPTIDE P directement ou par l'intermédiaire du Lien.
On citera aussi les chélates du document US2006/0018830 page 9 à 11 de la partie description [0150] à [0158].
Avantageusement dans le cadre de la présente invention, Ch est le DTPA ou le DOTA ou leurs dérivés.

Dans le cas de l'IRM, la relaxivité r₁ de ces chélates est typiquement de l'ordre de 4 à 20 s⁻¹ mMol⁻¹ Gd⁻¹ à un champ de 0,5 à 1,5 T. On rappelle que la relaxivité longitudinale r₁ d'un produit de contraste paramagnétique donne la mesure de son efficacité magnétique et permet d'apprécier son influence sur le signal enregistré. En imagerie médicale IRM, les produits de contraste modifient le temps de relaxation des protons, et l'augmentation de la relaxivité obtenue permet d'obtenir un signal plus élevé.

On entend dans la formule I par le terme liaison chimique, un groupe de liaison ou Lien L, c'est-à-dire un groupe chimique :
- permettant de relier le SIGNAL et le ou les PEPTIDE P
- n'ayant pas lui-même la fonction d'entité signal qui est assurée par le Signal
- n'ayant pas lui-même la fonction de ciblage qui est assurée par le PEPTIDE P

Le couplage de chélates avec des biovecteurs en particulier peptidiques est décrit dans l'art antérieur, et fait généralement intervenir une liaison chimique (Lien) telle que décrite dans le document WO01/60416. La structure et la nature chimique de la liaison chimique sont définies pour permettre le couplage chimique entre la partie peptidique du PEPTIDE P et le ou les chélates utilisés, et de manière à obtenir une affinité de la partie PEPTIDE P pour sa cible et une spécificité de la reconnaissance appropriée pour l'utilisation.

Un grand nombre de Lien peuvent être utilisés, dans la mesure où ils sont capables d'interagir avec au moins un groupe fonctionnel de biovecteur et au moins un groupe fonctionnel de chélate.
Avantageusement Lien représente :
a) un groupe de formule Q1 -1- Q2,
   dans laquelle Q1 et Q2 identiques ou différents représentent O, S, NH, CO₂, -NHCO, CONH, NHCONH, NHCSNH, SO₂NH- ou NHSO₂-,
   et 1 représente un groupe alkyle (avantageusement en C₁-C₁₀), alcoxyalkyle (avantageusement en C₁-C₁₀), alcényle (avantageusement en C₂-C₆), alcynyle (avantageusement en C₂-C₆), polyalkoxyalkylène, alkyle interrompu par un ou plusieurs squarate, par un ou plusieurs aryles, avantageusement phényle, ou par un ou plusieurs groupes choisis parmi -NH-, -O-, -CO-, -NH(CO)-, -(CO)NH-, -O(CO)-, ou -(OC)O- :
b) un groupe (CH₂)ₙ, (CH₂-CO-, -(CH₂)ₙNH-CO- avec n = 2 à 10, (CH₂CH₂O)_{q}(CH₂)ᵣ-CO-, (CH₂CHO)_{q}(CH₂)ᵣ-NH-CO- avec q = 1-10 et r=2-10. (CH₂)ₙ-CONH- , (CH₂)ₙ-CONH-PEG, (CH₂)ₙ-NH-, 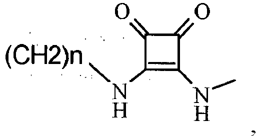 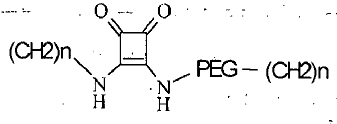 avec n=1 à 5 et avantageusement n=4 ou 5, HOOC-CH₂-O-(CH₂)₂-O-(CH₂)₂-O-CH₂-COOH ; HOOC-(CH₂)₂-CO₂-(CH₂)₂-OCO-(CH₂)₂-COOH; HOOC-CH(OH)-CH(OH)-COOH: HOOC-(CH₂)ₙ-COOH; NH₂-CH₂)ₙ-NH₂, avec n=1-20; NH₂-(CH₂)ₙ-CO₂H; NH₂-CH₂-(CH₂-O-CH₂)ₙ-CO₂H avec n=1 à 10.
   Parmi les linkers avantageux, on citera en particulier ceux des exemples de la présente demande :
   CH2 - phényle - NHCSNH - (CH₂O) _{1 = i à 3} 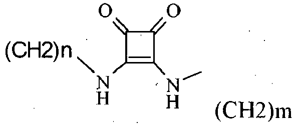 avec n=1 à 5 et m = 0 à 5 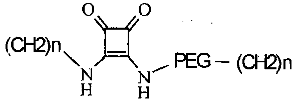 avec n = 1 à 5 et m = 0 à 5
   (CH2)n - CO avec n= 1 à 5
   (CH₂)₃ - squarate - (CH₂CH₂O)₂(CH₂)-CO
c) des liens décrits dans le brevet US 6 264 914, capables de réagir avec les groupes fonctionnels (du biovecteur et du chélate) amino, hydroxyle, sulfhydrile, carboxyle, carbonyle, carbohydrates, thioéthers, 2-aminoalcobols, 2-aminothiols, guanidinyle, imidazolyle, phénolique ; selon les définitions de ce document.
d) certains liens décrits dans le brevet US 6 537 520 de formule
   - (Cr₆r₇)_{g}-(W)ₕ-(Cr₆ₐr₇ₐ)_{g'}-(Z)ₖ-(W)_{h'}-(Cr₈r₉)_{g"}-(W)_{h"}-(Cr₈ₐr₉ₐ)_{g'"} avec :
      g+h+g'+k+h'+g"+h"+g"' différent de 0; avec les définitions identiques à celles de ce document colonne 8.
e) certains liens décrits dans le document WO 02/085908 (avec les définitions identiques à celles de ce document), par exemple une chaîne linéaire ou ramifiée de liaison organique choisie parmi :
   - CR6"'R7"'-, - (R6"')C=C(R7"')=, -CC-, -C(O)-, -O-, -S-, -SO₂-, -N(R3"')-, - (R6"')C=N-, -C(S)-, -P(O0(OR3"')- -P(O)-(OR3"')O-, avec R"'3 un groupe capable de réagir avec un azote ou un oxygène
   - une région cyclique (cycloalkyles divalents, hétérocycles divalents)
   - des polyalkylènes, polyalkylènes glycols
f) des liens du document WO03/011115 pages 124-125
g) des liens du document US 2006/0018830 (le Lien du demandeur correspondant au lien désigné N-O-P dans ce document US 2006/0018830)
   - liens comprenant au moins un acide aminé non alpha (page 12 à 15, tableau 1 de ce document)
   - liens comprenant au moins un acide aminé non alpha porteur d'un groupe cyclique (page 18 à 25, tableau 3 de ce document)
   - liens ne comprenant pas d'acide aminé
   - autres liens (page 27, 28 de ce document)

Le choix de Lien (structure et taille) pourra se faire notamment de manière à contrôler notamment la charge, la lipophilie, l'hydrophilie du produit de formule (I), pour optimiser le ciblage biologique, la biodistribution. On pourra en particulier utiliser des liens biodégradables in vivo, des liens PEG ou mini-PEG.
Le choix du lien est effectué de manière à ne pas altérer de manière gênante l'efficacité du composé de formule (I) selon l'invention, un test permettant de vérifier cette efficacité in vitro et in vivo étant présentés dans la description détaillée.

Selon un autre mode de réalisation, Signal représente une molécule fluorescente utilisée en imagerie optique. Parmi les molécules fluorescentes utili imagerie optique on citera notamment ceux de US2006/0018830 et notamment ceux cités page 11 et 12 paragraphe 1.B, avec des modalités précises d'imagerie décrites colonne 33 ([0259]) au paragraphe 6 (techniques et chromophores et fluorophores décrits en détail).

Selon un autre mode de réalisation, Signal représente une nanoparticule superparamagnétique revêtue d'une couche organique, avantageusement communément désignée SPIO ou USPIO (« ultra small particles of iron oxide »). Avantageusement, la nanoparticule comporte un noyau d'oxyde ou d'hydroxyde de fer, notamment de magnétite (Fe₃O₄), maghémite (γ-Fe₂0₃). On utilisera avantageusement une nanoparticule recouverte d'un revêtement bisphosphonate, avantageusement *gem*-bisphosphonate, décrite dans WO2004058275, la particule et la méthode de couplage entre le peptide et la nanoparticule étant détaillées dans les exemples de la présente demande. Les nanoparticules magnétiques utilisées sont des nanoparticules acides à base d'un composé du fer; et recouvertes par une couche comprenant un ou plusieurs composés *gem-*bisphosphonates identiques ou différents, la couche de recouvrement de la nanoparticule avant la formule (C) suivante:

T-L2-CH(PO₃H₂)₂ (C)

dans laquelle :
- Le lien L2 représente un groupement organique reliant la fonction T à la fonction *gem-*bisphosphonate -CH(PO₃H₂)₂ ;
- T représente une fonction chimique couplé au PEPTIDE P ou au Lien de la présente demande.
Dans un mode de réalisation particulier T-L2 représente le Lien du composé de formule (I).
La composition se présente sous forme d'une solution aqueuse stable de nanoparticules. Dans ces compositions, le taux de complexation (de la couche avec le peptide) du composé (C) sur les particules est supérieur à 50%, avantageusement à 70%, et de préférence supérieur à 80, 90, 95%. On préfère particulièrement que les particules magnétiques acides (p) soient complexées sur au moins 90% de leurs sites protonés par des composés de formule (C). Selon une variante, une partie des fonctions T de la couche est couplée à un PEPTIDE P, et une partie des fonctions T est couplée à un composé hydrophile notamment un composé porteur de groupes hydroxyle et notamment un composé hydrophile aminoalcool désigné AAG1AA28 décrit dans WO2004058275 (exemple 8) ou un groupe PEG.
Les particules magnétiques (p) possèdent un diamètre hydrodynamique compris entre 5 et 300 nm, de préférence entre 5 et 60 nm, encore de préférence entre 5 et 30 nm.
Le linker L2 permet de relier et/ou d'espacer la fonction gem-bisphosphonate et l'entité réactive T capable d'assurer le greffage covalent du PEPTIDE P (le biovecteur) sur la nanoparticule, par l'intermédiaire ou non du Lien..
A titre préféré, le lien L2 représente un groupement divalent.
De préférence, le lien L2 est choisi parmi :
- un groupe aliphatique : alicyclique : alicyclique aliphatique; aromatique ; aromatique aliphatique, lesdits groupes aliphatiques, alicycliques et aromatiques pouvant être éventuellement substitués par un groupe méthyle, hydroxy, méthoxy, acétoxy, amido, ou un atome d'halogène. avantageusement un atome de chlore, d'iode ou de brome ;
- un groupement -l₁ -NHCO-l₂ où l₁ et l₂, identiques ou différents, représentent un groupe aliphatique ; alicyclique : aromatique ; alicyclique aliphatique ou aromatique aliphatique, lesdits groupes pouvant être éventuellement substitués par un groupe méthyle, hydroxy, méthoxy, acétoxy, amido ou un atome de chlore, d'iode ou de brome.
Selon des réalisations préférées, L2 représente un groupe aliphatique substitué ou non, et plus préférentiellement un groupe -(CH₂)ₚ -, où p est un entier de 1 à 5, ou préférentiellement un groupe -(CH₂)ₙ-NHCO-(CH₂)ₘ- où n et m représentent un nombre entier de 0 à 5.
A titre de groupes T préférés, on peut citer notamment les groupes COOH, -NH₂, -NCS, -NH-NH₂, -CHO, alkylpyrocarbonyle (-CO-O-CO-alk), acylazidyle (-CO-N₃), iminocarbonate (-OC(NH)-NH₂), vinylsulfuryle (-S-CH=CH₂), pyridyldisulfuryle, (-S-S-Py), haloacétyle, maléimidyle, dichlorotriazinyle, halogène, les groupes -COOH et -NH₂ étant particulièrement préférés.
De préférence, T représente un groupe -COOH ou -NH₂ et L2 un groupe aliphatique substitué ou non, avantageusement un groupe -(CH₂)ₚ-, où p est un entier de 1 à 5.
On préfère tout particulièrement la couche de formule (C1) suivante :

HOOC-(CH₂)₂-CH(PO₃H₂)₂

Plusieurs composés de type nanoparticules d'oxyde de fer porteurs de PEPTIDE P sont décrits dans la description détaillée.
Dans l'utilisation d'un composé du formule générale (I) pour la préparation d'une composition diagnostique de maladies associées à l'apoptose et selon un autre mode de réalisation moins avantageux. Signal représente une nanoparticule lipidique comprenant au moins un chélate. Les nanoparticules lipidiques peuvent se trouver sous la forme d'émulsion nanoparticulaire, contenant ou non des perfluorocarbones, telle que celles décrites dans les documents WO 03/062198, US 5 958 371, US 5 080 885, US 6 403 056.
Les nanoparticules lipidiques peuvent être mises en suspension dans un milieu aqueux ou hydrophile. Ces nanoparticules ont un diamètre de l'ordre de 10 nm à 500 nm, notamment 20 à 250 nm. Les nanoparticules en émulsion peuvent comprendre ou être couplées avec un grand nombre de chélates, par exemple 10 000 à 100 000 chélates par nanoparticule. Les émulsions comprennent suffisamment de composés de formule (I) et donc de Peptide pour permettre la reconnaissance de la zone d'apoptose. On citera comme nanoparticules possibles des liposomes, unilamellaires ou multilamellaires, des micelles, des microgels, des gouttelettes d'huiles, des lipoprotéines, tels que HDL, LDL, IDL, VLDL, chylomicrons, des nanoparticules de fluorocarbone, des nanobulles, ou analogues dont la surface est lipophile.
Avantageusement le chélate est lipophile et attaché à la membrane de la nanoparticule.

De façon avantageuse le Lien du composé de formule (I) est suffisamment lipophile pour coupler le Peptide à la membrane de la nanoparticule lipidique, le PEPTIDE P étant suffisamment exprimé à la partie externe de la nanoparticule pour la reconnaissance spécifique de la cible apoptotique. Le Lien est par exemple un groupe lipophile tel qu'une chaîne alkylène en C10-C20, cette chaîne s'insérant dans la couche lipidique de la nanoparticule et permettant ainsi d'attacher le Peptide à la nanoparticule.

De nombreux chélates rendus lipophiles pour être associés à une membrane lipidique sont décrits en détail notamment dans les documents US 6 045 821, WO90/04943, WO 2006/100305. Selon des réalisations le chélate porte une longue chaîne lipophile (phospholipide par exemple) qui vient s'insérer dans la membrane de la nanoparticule lipidique (liposome, micelle, nanoémulsion). De même le PEPTIDE P porte avantageusement une chaîne lipophile (le Lien) qui vient s'insérer dans la membrane de la nanoparticule lipidique.
Selon des réalisations avantageuses, la nanoparticule lipidique inclut des perfluorocarbones tels que décrits dans US 5,958,371, l'émulsion liquide contenant des nanoparticules comportant un perfluorocarbone à point d'ébullition assez élevé (par exemple entre 50 et 90 °C) entouré d'un revêtement composé d'un lipide et/ou d'un surfactant. Le surfactant est capable de se coupler directement à un biovecteur de ciblage ou d'inclure un composé intermédiaire lié de manière covalente au biovecteur, le cas échéant à l'aide d'un agent de liaison chimique.

Différentes émulsions de perfluorocarbone sont rappelées dans le document US 6 676 963 (perfluorodecalin, perfluorooctane, perfluorodichlorooctane, bromure de perfluoro-n-octyle, perfluoroheptane, perfluorodécane, perfluorocyclohexane, perfluoromorpholine, perfluorotripropylamine, perfluorotributylamine, perfluorodiméthylcyclohexane, perfluorothméthylcyclohexane, éther de perfluorodicyclohexyle, perfluoro-n-butyltétrahydrofurane).
On utilise habituellement comme phospholipides formant la membrane des nanoparticules les composés suivants : phosphatidylcholine dioléoylphosphatidylcholine, dimyristoylphosphatidylcholine, dipalmitoylphosphatidylcholine. distéaroylphosphatidylcholine, phosphatidyléthanolamine.
De telles compositions sont décrites par exemple dans US 5,989,520 et US 5,958,371, comme rappelé dans le document US 20040248856 qui cite notamment des composés perfluorocarbone : perfluorodécalin, perfluorooctane, perfluorodichlorooctane, bromure de perfluoro-n-octyle, perfluoroheptane et analogues.
Selon des réalisations avantageuses, on utilisera pour préparer des agents de contraste selon l'invention des méthodes et des compositions lipidiques appropriées rappelées dans US 6 010 682, notamment pour ce qui concerne la description détaillée de la composition lipidique, et de la préparation de liposomes, de micelles, d'émulsions.
On rappelle que les émulsions sont des mélanges lipidiques hétérogènes obtenues de manière appropriée par agitation mécanique et/ou addition d'agents émulsifiants. Par exemple, on mélange mécaniquement les chélates rendus lipophiles à des solvants organiques tels que le chloroforme. Après évaporation du solvant, les lipides sont remis en suspension en milieu aqueux tel que PBS pour obtenir une émulsion qui subit ensuite typiquement une sonication et une microfluidisation. Les émulsions obtenues peuvent être lyophilisées avec le cas échéant utilisation d'agents anti agglutination.
Pour formuler l'émulsion d'agent de contraste paramagnétique souhaité, on utilise typiquement 1 à 75% en poids de composé chélate lipophile par rapport aux ingrédients totaux de l'émulsion. La composition formant l'agent de contraste est administrée de préférence en intravasculaire, selon le patient examiné, par exemple à raison de 0,1 mg à 1 g de composé chélate lipophile et de 1 à 50 micromoles d'ion de métal paramagnétique par kg de patient.
Les compositions lipidiques obtenues sont le cas échéant formulées à l'aide d'additifs rappelés dans US 6 010 682, notamment pour une administration par injection intraveineuse. On citera notamment le dextrose, le chlorure de sodium, des antimicrobiens.
Avantageusement grâce aux compositions selon l'invention on peut obtenir une augmentation de la relaxivité par ion. On obtient typiquement les caractéristiques suivantes, pouvant varier selon les compositions précises des émulsions et leur procédé de préparation :
- index de polydispersité : 0,2 à 0,3
- [Gd³⁺] = 2 à 10 mM de préférence 3 à 7 mM
- concentration en particules : 50 à 100 nM
- r1 (mM⁻¹s⁻¹Gd⁻¹) : 5 à 40, de préférence 10 à 40
- r2 (mM⁻¹s⁻¹Gd⁻¹) 20 à 40
- r1 (mM⁻¹s⁻¹particule-1) : 10⁶ à 4x10⁶
- nombre de biovecteurs : 50 à 1000, notamment 100 à 300

L'invention concerne selon un autre aspect les produits de contraste pour l'IRM comprenant un composé de formule (I) tel que décrit précédemment dont l'ion de métal paramagnétique M est de nombre atomique 21-29, 42-44 ou 58,70, de préférence de gadolinium. Les métaux paramagnétiques M incluent les lanthanides de nombre atomique 58-70 et les métaux de transition de nombre atomique 21-29, 42 or 44, par exemple scandium, titane, vanadium, chrome, manganèse, fer, cobalt, nickel, cuivre, molybdène, ruthénium, cérium, praseodymium, neodymium, prométhium, samarium, europium, gadolinium, terbium, dysprosium, holmium, erbium, thulium, et ytterbium. On préfère particulièrement les éléments Gd(III), Mn(II), europium, dysprosium, avantageusement M est choisi parmi Gd, Mn, Fe, Dy et Tm. L'invention concerne selon un autre aspect les produits de contraste pour l'imagerie aux rayons X ou à la CT, comprenant un composé (I) tel que décrit précédemment dont l'ion de métal lourd M est de nombre atomique 21-31, 39-50, 56-80, 82, 83 ou 90.
L'invention concerne selon un autre aspect des produits radiopharmaceutiques, comprenant un composé de formule (I) tel que décrit précédemment dont le chélate est couplé avec un radionucléide ou un radiohalogène connu de l'homme du métier, typiquement le gadolinium, le technétium, le chrome, le gallium, l'indium, l'ytterbium, le rhénium, le lanthanium, l'yttrium, le dysprosium, le cuivre ou analogue. Les radionucléides incluent les formes radioactives des éléments Sm, Ho, Y, Pm, Gd, La, Lu, Yb, Sc, Pr, Tc, Re, Ru, Rh, Pd, Pt, Cu, Au, Ga, In, Sn, Cr, Pb, notamment ⁹⁹Tc, ¹¹⁷Sn, ¹¹¹In, ⁹⁷Ru, ⁶⁷Ga, ⁶⁸Ga, ⁸⁹Zr, ¹⁷⁷Lu, ⁴⁷Sc, ¹⁰⁵Rh; ¹⁸⁸Re, ⁶⁰Cu, ⁶²Cu, ⁶⁴Cu, ⁶⁷Cu, ⁹⁰Y, ¹⁵⁹Gd, ¹⁴⁹Pr, ¹⁶⁶Ho ; Le ⁶⁸Ga est le moins avantageux dans le cadre de la présente invention. La préparation de composés utilisables comme radiopharmaceutiques notamment en utilisant le technétium ⁹⁹Tc est rappelée dans US 2006/0018830 page 32 paragraphe 4, ces techniques étant décrites dans ce document pour des composés comprenant un chélate couplé à un peptide de ciblage de la gastrine.
Pour des indications en radiothérapie, le couplage de macrocycles de type DOTA, la sélection de nucléides appropriés, la préparation des composés radiothérapeutiques est rappelée dans US 2006/0018830 colonne 35 et 36.

La présente invention concerne en outre une composition comprenant au moins un composé de formule générale (I) tel que décrit précédemment et un excipient pharmaceutiquement acceptable, avantageusement destinée à une administration par voie parentérale. Elle concerne de plus un procédé de préparation d'une telle composition comprenant l'addition d'un composé de formule générale (I) tel que défini précédemment à un milieu injectable comprenant l'excipient pharmaceutiquement acceptable.

L'invention concerne l'utilisation d'une composition selon la présente invention pour le diagnostique d'une pathologie associée à l'apoptose. Les compositions diagnostiques et radiopharmaceutiques selon l'invention peuvent être utilisées comme décrit dans les demandes US 2002/0090342, US 2002/0098149, WO 02/055111 pour des indications anticancéreuses. L'invention concerne de plus les composés de formule générale (I) tels que définis précédemment pour leur utilisation en tant qu'agent de diagnostique de maladies associées à l'apoptose.
L'invention concerne aussi l'utilisation des composés décrits précédemment pour la préparation d'une composition diagnostique ou radiopharmaceutique destinée au diagnostique et/ou au traitement de maladies associées à l'apoptose, avantageusement choisies parmi les maladies neurodégénératives chroniques, les maladies ischémiques et les pathologies inflammatoires.

Elle concerne enfin l'utilisation d'un peptide ciblant l'apoptose de formules (3) telles que définies ci-dessus pour la préparation d'une composition de diagnostique de maladies associées à l'apoptose.

Le cas échéant les composés de formule générale (I) du demandeur seront utilisés en tant qu'agent de diagnostique, ou en tant qu'agent de traitement thérapeutique au niveau de zones d'apoptose, ou en tant qu'agent de diagnostique et de traitement thérapeutique, ou de suivi diagnostique de l'efficacité thérapeutique. Le cas échéant le composé sera co-administré simultanément ou de manière différée avec d'autres agents diagnostiques et/ou thérapeutiques ciblant l'apoptose. L'invention concerne aussi une méthode d'imagerie comprenant la synthèse d'un composé comprenant un métal paramagnétique selon l'invention, capable de cibler une zone pathologique, son administration à un patient, l'imagerie par IRM. L'invention concerne aussi une méthode d'imagerie comprenant la synthèse d'un composé radiopharmaceutique selon l'invention, capable de cibler une zone pathologique, son administration à un patient, l'imagerie par scintigraphie gamma SPECT ou planaire, ou tomographie par émission de positrons.
Pour un diagnostique en IRM, l'administration intraveineuse par injection habituellement en solution saline, se fait typiquement à une dose d'ion métallique de 0,001 à 1,5 mmole/kg de poids corporel, par exemple de 1 à 500 µmol Gd/kg.
Pour un diagnostique radiopharmaceutique, l'administration intraveineuse par injection habituellement en solution saline, se fait typiquement à une dose de 1 à 100 mCi pour 70 kg de poids corporel, de préférence de 5 à 50 mCi, avec une imagerie de diagnostic par exemple 30 à 180 minutes après l'injection pour le ⁹⁹Tc.
Pour une utilisation comme agents de contraste aux rayons X, la concentration en atome lourd est typiquement de 0,1 M à 5 M, avec des concentrations par administration intraveineuse de l'ordre de 0,5 à 1,5 mmol/kg.

L'invention concerne selon un autre aspect l'utilisation d'un composé de formule (I) tel que décrit précédemment pour la préparation d'une composition destinée à l'imagerie optique.
Des exemples d'administration de compositions pour l'imagerie médicale sont décrits dans l'art antérieur, par exemple dans le document WO 0226776 et US 2006/0018830 colonne 36 ([0282]) paragraphe 8 (dosages et additives).
Des véhicules pharmaceutiquement physiologiquement acceptables permettant de former des compositions diagnostiques (produits de contraste) comprenant les composés décrits précédemment sont connus de l'art antérieur. On utilisera par exemple des sels (sodium, calcium, méglumine), des agents de contrôle du pH (acide acétique, citrique, fumarique, des antioxydants.

L'invention concerne aussi un procédé de préparation de composés comprenant le couplage d'un Peptide avec au moins un chélate. Plusieurs méthodes générales de préparation de composés de formule (I) décrites dans le US2006/0018830 (Bracco) sont applicables en utilisant Peptide à la place des peptides de ces documents. Ces méthodes choisies en fonction notamment du chélate choisi sont rappelées dans 2006/0018830 colonne 37 ([0288] à ([0291]) (« general préparation of compounds ». et « alternative préparation of the compounds via segment coupling »), par exemple les méthodes SPPS et FMOC.
L'invention concerne aussi une méthode de diagnostique ou de traitement radiopharmaceutique comprenant l'administration d'un composé (I), la réalisation d'un examen d'imagerie à l'aide d'un équipement approprié, et l'analyse des résultats.
L'invention couvre sauf indication contraire, toutes les formes chirales, diastéréoisomères, racémiques, notamment cis-trans, L-D des composés de formule (I) décrits.

Le demandeur a aussi étudié les possibilités d'association d'un PEPTIDE P couplé à plusieurs chélates dans le composé de formule (I). Le demandeur a par ailleurs étudié des composés de formule (I) présentant un assemblage entre un ou plusieurs Peptides du composé de formule (I) ciblant l'apoptose, et un ou plusieurs chélates ; et de manière à ce que l'accès à la cible ne soit pas gêné malgré la présence du ou des chélates. Par exemple, le chélate est espacé du ou des PEPTIDES P par le Lien d'une taille suffisante et d'une structure chimique telles que la reconnaissance du ou des peptides par leur cible n'est pas altérée.
Parmi les associations biovecteurs (peptides ou éventuels d'autres biovecteurs) /chélates, on peut citer notamment :
- un peptide biovecteur central relié à plusieurs chélates identiques ou différents
- un chélate central relié à plusieurs peptides identiques ou différents
- un premier ensemble [peptide porteur de chélate(s)] couplé par l'intermédiaire d'un lien hydrophobe ou hydrophile à un second ensemble [peptide porteur de chélates(s)], s'écrivant par exemple:
   Ch2-peptide1-Lien-peptide2-Ch2 avec Ch représentant des chélates identiques ou différents et peptide 1 et peptide2 représentant des Peptides identiques ou différents
- un ensemble peptide1-(Lien porteur de Ch)-peptide2-Ch
On utilisera par exemple la méthode de construction de composés multimériques décrite dans US2005/0100963 (WO2006/031885 page 66 ligne 25 à page 69 ligne 30) dans le cas de peptides ciblant des récepteurs KDR (par exemple à l'aide de la méthode 13 des exemples : « préparation of homodimers and heterodimers »), mais en utilisant le PEPTIDE P (on remplacera, par exemple les peptides des composés des figures 44 à 47 du US2005100963 par des PEPTIDES P). Le composé peut ainsi avantageusement comprendre un peptide couplé à plusieurs chélates, ou un chélate couplé à plusieurs, peptides identiques ou différents: L'invention concerne aussi des composés mixtes comprenant en plus du PEPTIDE P au moins un autre biovecteur ciblant l'apoptose, le biovecteur étant soit un autre peptide soit un autre biovecteur mais non peptidique. On pourra le cas échéant coupler la partie peptide ou la partie chélate à des groupements chimiques permettant de favoriser la biodistribution, la durée de vie du produit dans le sang.
La spécificité du produit fait référence à son affinité spécifique pour au moins un marqueur de l'apoptose ou de tout trouble pathologique associé, la spécificité de la liaison exprimée typiquement par des constantes Kd et Ka, la valeur Kd pour les marqueurs cibles étant inférieures à 10 µM, de préférence inférieure à 1 µM.

Parmi les sels pharmaceutiquement acceptables, on citera notamment des sels de cations de bases inorganiques (potassium, sodium, calcium, magnésium...), des cations de bases organiques (éthanolamine, diéthanolamine, morpholine, glucamine, N-méthylglucamine, N, N-diméthylglucamine...), des anions d'acides inorganiques (notamment des chlorures, bromures, iodures, sulfates...), des anions d'acides organiques (acétate, succinate, citrate, fumarate, maléate, oxalate, trifluoroacétate...), des ions d'acides aminés (taurine, glycine, lysine, arginine, ornithine, acide aspartique, acide glutamique...).

Le demandeur a démontré l'utilisation très avantageuse de composés incorporant des peptides selon l'invention, en particulier en IRM, pour plusieurs catégories de troubles dûs à l'apoptose : maladie vasculaire (apoptose au niveau de zones à plaques d'athérome), altération de tissus hépatiques (apoptose hépatique), altération de tissus nerveux (apoptose neuronale à l'origine de maladies cérébrales dont la maladie de Parkinson en particulier).

Il est rappelé qu'il existe un écart très important entre l'efficacité-potentielle in vitro d'un biovecteur de ciblage, et son efficacité réelle in vivo une fois couplé à une entité signal. Comme illustré plus loin, le demandeur a démontré notamment l'efficacité de produits pour IRM comprenant un chélate lié au peptide P notamment LIKKPF, et de produits pour IRM de type USPIO (particule d'oxyde de fer) lié au peptide P notamment LIKKPF.
Ainsi quand bien même un peptide serait connu de l'art antérieur, l'identification de son utilité dans un mécanisme d'apoptose par son ciblage en particulier de la phosphatidylsérine, parmi le nombre extrêmement important de cibles biologiques possibles, est loin d'être évidente. De plus il n'est nullement évident que cette cible biologique identifiée, des composés couplés (peptide P
- entité signal) permettent de résoudre les problémes techniques résolus par le demandeur, notamment :
- la conservation de l'affinité in vivo pour le site reconnaissance biologique, malgré l'encombrement stérique et la modification possible de conformation in vivo du fait du couplage à une entité signal
- la possibilité de couplage chimique avec les entités signal : le couplage avec les dérivés PCTA en particulier a impliqué la mise au point d'une synthèse chimique dédiée
- la stabilité physico-chimique in vivo qui constitue une limitation majeure pour de nombreux peptides
- la capacité d'être reconnu en imagerie in vivo, et cela en particulier en IRM, technique dont le niveau de sensibilité est près de 1000 fois inférieur à l'imagerie PET.

Au global, il n'était donc absolument pas évident pour l'homme du métier :
- d'une part de sélectionner les peptides objet de la présente invention
- d'autre part que les produits intégrant ces peptides soient in vivo effectivement efficaces.

Dans la description détaillée qui suit, on décrit des peptides dont on a montré l'efficacité avec des variantes de réalisation sur différentes entités signal (PARTIE I) et les résultats biologiques (PARTIE II).
La figure 1 représente le test de compétition avec l'annexine V.
La figure 2 représente la mesure de l'affinité du peptide LIKKPF (SEQ ID No 11).
La figure 3 représente la mesure de l'affinité du peptide LIKKPF (SEQ ID No 11) couplé à un USPIO.
La figure 4 illustre des tests sur cellules apoptotiques réalisés avec un produit USPIO-LIKKPF. La figure 5 illustre l'affinité d'un produit DTPA-LIKKPF avec les cellules apoptotiques.
La figure 6 illustre le suivi dynamique du signal IRM du foie apoptotique de souris avec un produit DTPA-LIKKPF.
La figure 7 montre des images IRM sur souris ApoE obtenues avec un produit DTPA-LIKKPF. Là figure 8 représente le rehaussement quantitatif correspondant.

### EXEMPLES PARTIE I : préparation des composés incluant des PEPTIDES P.

### Généralités

M : concentration molaire (mol/l).
M/z : masse sur charge déterminée par spectrométrie de masse.
ES⁺ : électrospray mode positif.
ES⁻: électrospray mode négatif.
kD : unité de masse moléculaire (kiloDalton)
CCM : Chromatographie sur Couche Mince
Z ave : diamètre hydrodynamique mesuré par PCS

### Dosage du Fer total :

Le fer est dosé par spectroscopie d'absorption atomique (Spectrophotomètre VARIAN AA10) après minéralisation par HCl concentré et dilution par rapport à une gamme étalon d'ions ferrique (0, 5, 10, 15 et 20 ppm)

### Taille des particules :

### Diamètre hydrodynamique de la particule greffée (Z ave) :

Déterminé par PCS (appareil Malvern 4700, laser 488 nm à 90°) sur un échantillon dilué à - 1 millimolaire avec de l'eau PPI filtrée sur 0.22 µm.
PCS = Photon Correlation Spectroscopy = Technique par Diffusion de Lumière Dynamique - Référence : R. Pecora dans J. of Nano. Res. (2000), 2, p 123-131.

### Analyses structurales :

Par spectroscopie de masse (appareil MICROMASS VG Quattro II) avec une source Electrospray.

### Mesures de relaxivité :

Les temps de relaxation T1 et T2 ont été déterminés par des procédures standards sur un appareil Minispec 120 (Bruker) à 20 Mhz (0,47T) et 37°C . Le temps de relaxation longitudinal T1 est mesuré en utilisant une séquence d'Inversion Récupération et le temps de relaxation transverse T2 est mesuré par une technique CPMG.
Les vitesses de relaxation R1 (=1/T1) et R2(=1/T2) ont été calculées pour différentes concentrations en métal total ( variant de 0.1.10⁻³ à 1.10⁻³ mole/L) en solution aqueuse à 37°C.

La corrélation entre R1 ou R2 en fonction de la concentration est linéaire, et la pente représente la relaxivité r1 (R1/C) ou r2 (R2/C) exprimées en (1 / seconde) x (1/ mmol/L) soit (s⁻¹.mM⁻¹).
Les nanoparticules ont été préparées selon les méthodes décrites dans le brevet WO2004/058 275 (US 2004/253181) exemple 8 et 9 pour la préparation des solutions colloïdales de particules magnétiques et exemples 10 à 12 pour la complexation des particules magnétiques par un revêtement gem-bisphosphonate de l'exemple 1 de WO2004/058 275.

### Exemple 1 : Couplage des peptides sur la particule d'oxyde de fer

### (produit PEG - USPIO - PEPTIDE P)

Les séquences peptidiques d'intérêt sont présentées dans le tableau suivant :

| N° | Séquence du PEPTIDE P |
|---|---|
| 1* | Asp-Ala-His-Ser-Phe-SerOH |
| 2* | Leu-Ile-Lys-Lys-Pro-Phe-OH (SEQ ID No 11) |
| 3 | Pro-Gly-Asp-Leu-Ser-Arg-OH |
| 4* | Gly-Asp-Ala-His-Ser-Phe-SerOH |
| 5* | γ-Abu- Asp-Ala-His-Ser-Phe-SerOH |
| 6* | 8-Amino-3,6-dioxaoctanoyl- Asp-Ala-His-Ser-Phe-SerOH |
| 7* | 8-Amino-3,6-dioxaoctanoyl-Leu-Ile-Lys-Lys-Pro-Phe-OH |

| | |
|---|---|
| * à titre de comparaison | |

### Couplage du peptide:

30 ml de nanoparticules, [Fe] = 0,338 mol sont ultrafiltrés et agités à température ambiante, le pH est égal à 7,29. Le peptide N°2 (LIKKPF (SEQ ID No 11)), protégé sous forme de trifluoroacétamide sur les fonctions amines latérales des lysines, est dissout dans 1 ml d'eau (26,6 mg) et la solution obtenue est ajoutée progressivement en même temps que l'EDCI (25 mg). En fin d'ajout le pH est égal à 6,55. Le mélange est agité une nuit à température ambiante. Le pH est ajusté à 7 avec NaOH (0,1 N) et la solution est filtrée sur 0,22 µm (filtres Durapore Millipore) puis ultrafiltrée sur une membrane de 30 kD (éliminer - 1 1 de filtrat). Volume final 30 ml (solution A).
Les peptides 1, 3, 4, 5, 6 et 7 sont couplés selon le même mode opératoire.

### Couplage de l'amine hydrophile

24 ml de la solution obtenue précédemment (solution A) sont agités à température ambiante et une solution de 1,88g de l'amine polyhydroxylée (préparée selon le mode opératoire décrit dans le brevet EP 0 922 700 A1) est ajoutée dans 5 ml d'eau. Le pH est ajusté à 8 avec une solution d'HCl et 600 mg d'EDCI sont introduits à température ambiante. Le milieu est agité une nuit, le pH est ajusté à 7,5 et la solution est filtrée sur filtre de porosité 0,22µ puis ultrafiltrée sur une membrane de 30 kD. Volume final 20 ml (solution B).

### Déprotection des Lysines:

La solution précédente (solution B) est agitée à température ambiante et le pH est ajusté à 10 avec une solution de NaOH. Après 6h d'agitation, la solution est filtrée sur filtre de porosité 0,22 µ puis ultrafiltrée sur une membrane de 14 kD.

### Couplage des amines polyéthylèneglycol, PEG-NH₂

L'amino PEG-NH₂ 750 (O-(2-aminoéthyl)-O'-méthylpolyéthylèneglycol 750 est obtenu chez FLUKA®). L'aminoPEG 350 est préparé selon la séquence réactionnelle suivante selon des modes opératoires décrits dans la littérature.

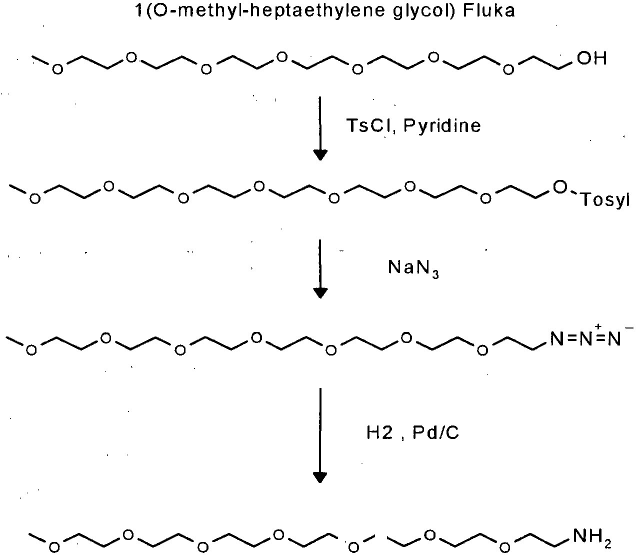

Les aminoPEG suivants sont disponibles chez les fournisseurs :

| Composé | Poids moléculaire | Nom commercial | fournisseur |
|---|---|---|---|
| 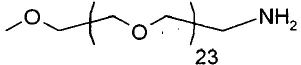 | 108.8 | m-dPEG₂₄ amine | Quanta Biodesign® |
| 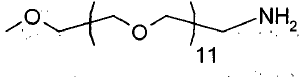 | 559 | m-dPEG₁₂ amine | Quanta Biodesign® |
| 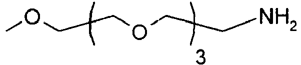 | 207 | m-dPEG₄ amine | Quanta Biodesign® |
| 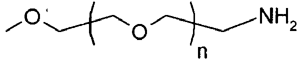 | 2000Da | mPEG-NH2 | Nektar® |

24 ml de la solution USPIO-peptide protégé (solution A) sont agités à température ambiante et une solution de PEG 750-NH2 (1,57 g) dans 5 ml d'eau est ajoutée. Le pH est ajusté à 8 avec une solution d'HCl et 1g d'EDCI est ajouté en deux portions égales. Le mélange est agité une nuit, le pH est ajusté à 7,5 et la solution est filtrée sur un filtre de 0,22µ puis ultrafiltrée sur une membrane de seuil de coupure 30 kD ; volume final 20 ml (solution C).
Les autres aminoPEG sont couplés selon le même mode opératoire au départ de la solution A.

### Caractérisation:

**A. PEG750 - USPIO -peptide N°2**
   concentration 110 mM de fer
   PCS : 26 nm
   r2/r1 (20 MHz 37°C): 72,95/28,12 = 2,59
   r2/r1 (60 MHz, 37°C): 71,81/12,59 = 5,70
**B. PEG2000- USPIO - peptide N°2**
   Concentration 20,96 mM de fer
   PCS : 40 et 150 nm
   r2/r1 (20 MHz, 37°C): 273,46/36,66 = 7,46
   r2/r1 (60 MHz, 37°C): 312,79/16,28 = 19,21
**C. PEG350- USPIO -peptide N°2**
   Concentration 142,59 mM de fer
   PCS : 25 et 90 nm
   r2/r1 (20 MHz, 37°C): 101,54/33,90 = 2,99
   r2/r1 (60 MHz, 37°C): 104,45/15,37 = 6,79

### Exemple 2 : couplage des peptides sur des chélates de gadolinium

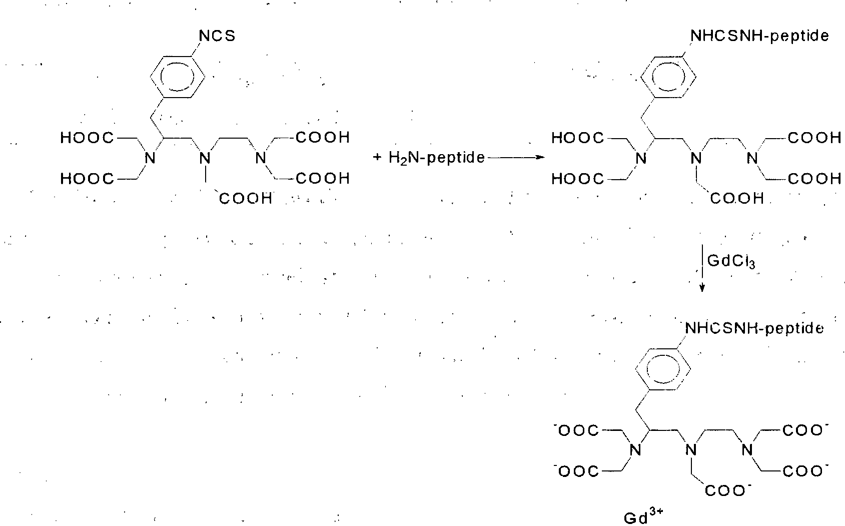

### Séquence des peptides couplés:

| N° | Séquence | Quantité mise en jeu |
|---|---|---|
| 1* | Asp-Ala-His-Ser-Phe-SerOH | 33 mg |
| 3 | Pro-Gly-Asp-Leu-Ser-Arg-OH | 32 mg |

| | | |
|---|---|---|
| * à titre de comparaison | | |

### Condensation des peptides et complexation:

A une solution de peptide dans 2 ml d'eau est ajouté goutte à goutte le composé Bz-NCS-DTPA (Macrocyclics ®) dissous dans du DMF. Le pH du mélange réactionnel est ajusté à 10 avec une solution de NaOH et la solution est agitée pendant 48h. Le pH est ensuite ajusté à 7, et la solution est percolée sur une colonne de silice RPC18. Les fractions contenant le composé d'addition sont recueillies. Le ligand est ensuite complexé avec un équivalent de GdCl₃.6H₂O pendant 6h.

### Spectres de masse ESI :

Peptide N°3-DTPAGd: [M+H]⁺ : 1184
Peptide N°1-DTPAGd: [M+H]⁺ : 1204
Relaxivités mesurées à 20 MHz (37°C) :
Peptide N°3-DTPAGd: 6.7 s⁻¹mM⁻¹
Peptide N°1-DTPAGd: 6.8 s⁻¹mM⁻¹

### Couplage d'un peptide protégé sous forme de trifluoroacétamides latérales:

| | | |
|---|---|---|
| 7* | 8-Amino-3,6-dioxaoctanoyl-Leu-Ile-Lys(tfa)-Lys(tfa)-Pro-Phe-OH | 350 mg |

| | | |
|---|---|---|
| * à titre de comparaison | | |

Le peptide (350 mg, 0.323 mmol) est dissous dans 2 ml d'eau et le composé polycarboxylé (NCS-Bz-DTPA 420 mg, 0,646 mmol, 2 équivalents) en solution dans l'eau est ajouté goutte à goutte. Le pH du mélange réactionnel est ajusté à 10 avec une solution de NaOH et la solution est agitée pendant 48h. Le pH est réajusté à 7, et la solution est percolée sur une colonne de silice RPC 18. Les fractions contenant le produit de condensation sont concentrées et le ligand est complexé avec un équivalent de GdCl₃.6H₂O (0,235 mmol) pendant 6h.

### Caractérisation:

### Spectre de masse du complexe de gd

| Complexes | r₁ à 0,47 T (s⁻¹.mM) | r₁ à 1,4 T (s⁻¹.mM⁻¹) |
|---|---|---|
| Gd - DTPA - R826 | 9,6 | 10,1 |

| | | |
|---|---|---|
| *Relaxivité du complexe de Gd* | | |

### Exemple 3 : synthèse d'un chélate bifonctionnel dérivé du DOTA

### Etape 1 : Ester benzylique de l'acide 5-(1,3-Dioxo-1,3-dihydro-isoindol-2-yl)-2-(1,4,7,10tétraaza-cyclododèc-1-yl)-pentanoïque

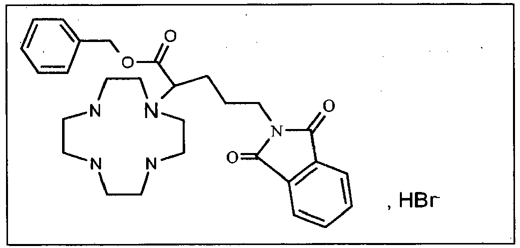

55 g de cyclen base (320 mmol) sont dissous dans 550 mL de CH₃CN, auxquels sont ajoutés goutte à goutte 119,8 g de dérivé bromé (2-Bromo-5-(1,3-dioxo-1,3-dihydro-isoindol-2-yl)-pentanoïque acide benzyle ester; 288 mmol) dissous dans 550 mL de CH₃CN Le milieu est agité à température ambiante 1 nuit. Le précipité est filtré et lavé abondamment à l'acétonitrile. Obtention de 138 g de produit sous forme d'une poudre blanche (rendement corrigé 81,3 %). CCM : CH₂Cl₂/ MeOH / NH₄OH à 25% (80/40/3)
Révélation UV et CuSO₄
Rf: 0,3

### Etape 2 : Ester benzylique de l'acide 5-(1,3-Dioxo-1,3-dihydro-isoindol-2-yl)-2-(4,7,10-tris-éthoxycarbonylméthyl-1,4,7,10tétraaza-cyclododéc-1-yl)-pentanoïque

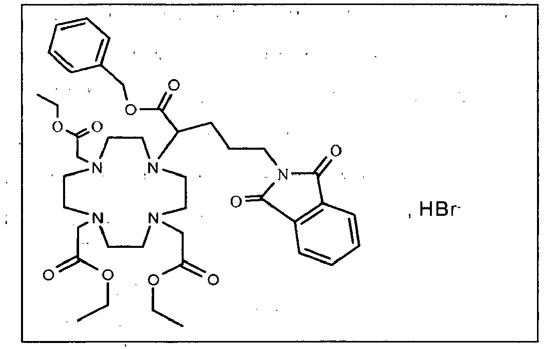

A une solution de 59.1 g d'ethylbromoacétate (Aldrich®, 358 mmol) dans le CH₃CN (1,11) sont additionnées 60 g du composé obtenu à l'étape 1 (102 mmol) et 50,1 g de Na₂CO₃ (464 mmol). Le milieu réactionnel est chauffé à 80°C sous couverture d'argon pendant une nuit. Après élimination du précipité, le filtrat est concentré et lavé abondamment au CH₃CN. Le produit est cristallisé dans CH₃CN par ajout goutte à goutte d'Et₂O. Obtention de 89,8 g de produit sous forme d'un solide blanc (rendement corrigé 100 %).
CCM : CH₂Cl₂ / MeOH (9/1)
Révélation UV et KMnO₄ ; Rf : 0,4

### Etape 3 : Acide 5-Amino-2-(4,7,10-tris-carboxyméthyl-1,4,7,10tétraaza-cyclododéc-1-yl)-pentanoïque

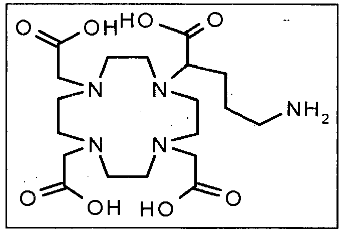

Dans le réacteur de 5 litres, on chauffe au reflux une solution de 54 g de composé obtenu à l'étape 2 (64 mmol) dans avide chlorhydrique 37 % (1,81), pendant une nuit. Après refroidissement et filtration, le filtrat est concentré et purifié sur silice silanisée (élution à l'eau). Après évaporation sous pression réduite, le produit est lavé à l'éther. Obtention de 45 g de produit sous forme d'un solide blanc. Le produit est désalifié par passage sur résine OH⁻. On isole 30 g de produit sous forme de cristaux blancs (rendement 100 %).
HPLC : Hypercarb® 5µ, 200X4.6, 250A ; Solvant A : acide sulfurique 0,037 N
Solvant B : CH₃CN; Détection UV à 201 nm ; Tr : 18 min.

### Etape 4 : Complexe de gadolinium de l'acide 5-Amino-2-(4,7,10-tris-carboxyméthyl-1,4,7,10tétraaza-cyclododéc-1-yl) -pentanoïque

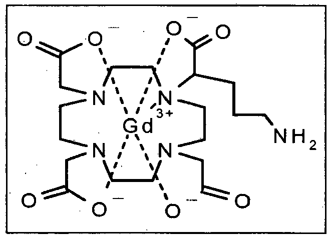

7,2 g du composé obtenu à l'étape 3(16 mmol) sont dissous dans 70 mL d'eau et le pH est ajusté à 5,5 par ajout d'acide chlorhydrique 6 N. On ajoute 2,9 g de Gd₂O₃ (8 mmol), et on chauffe à 80°C le milieu réactionnel. Le pH de la solution augmente régulièrement, et doit être maintenu entre 5,2 et 5,7 par ajout goutte à goutte d'acide chlorhydrique 6 N. Après deux heures, le pH se stabilise à 5,7. Le léger trouble est filtré sur filtre Whatman® et le filtrat est concentré. Obtention de 11,1 g de produit sous forme de paillettes blanches (rendement corrigé 100%).
HPLC : Hypercarb® 5µ, 200X4,6, 250Â : Solvant A : acide sulfurique 0,037 N
Solvant B : CH₃CN ; Détection UV à 201 nm ; Tr : 10 min.

### Etape 5 : Complexe de gadolinium de l'acide 5-(2-Ethoxy-3,4-dioxo-cyclobut-1-énylamino)-2-(4,7,10-tris-carboxyméthyl-1,4,7,10tétraaza-cyclododéc-1-yl)-pentanoïque

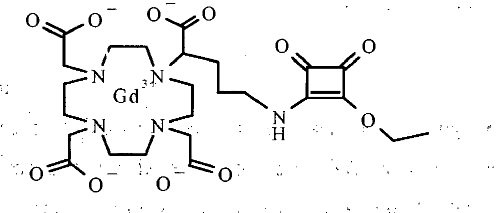

8 g de composé obtenu à l'étape 4 sont séchés par distillation azéotropique au toluène, puis mis en suspension dans 90 ml de DMSO anhydre sous couverture d'argon. 2.8 ml de Et₃N séchée sur tamis (1,7 éq) et 5 g de diéthylsquarate (Aldrich®, 2,5 éq.) sont ensuite ajoutés. Le milieu est agité à température ambiante sous couverture d'argon pendant 1 heure. Le mélange est précipité dans 600 ml d'éther. Le solide obtenu est filtré, puis lavé au dichlorométhane. Après filtration et séchage, 7,5 g d'un solide blanc (rendement de 81,5 %)sont récupérés.
HPLC : Symmetry C18, 5µ, 250X4,6, 100Å
A : eau TFA, pH = 2, 7 ; B : CH₃CN ; Détection à 201 et 254 nm ; Tr : 19,8 min.

### Exemple 4 : Couplages des peptides

| N° | Séquence | P M |
|---|---|---|
| 1* | Asp(tBu)-Ala-His(trt)-Ser(tBu)-Phe-Ser(tBu)OH | 1073,31 |
| 2* | Leu-Ile-Lys(Boc)-Lys(Boc)-Pro-Phe-OH | 945,22 |
| 3 | Pro-Gly-Asp-(tBu)-Leu-Ser(tBu)-Arg(Pbf)-OH | 1008,25 |
| 4* | Gly-Asp(tBu)-Ala-His(trt)-Ser(tBu)-Phe-Ser(tBu)QH | 1130,36 |
| 5* | γ-Abu-Asp(tBu)-Ala-His(trt)-Ser(tBu)-Phe-Ser(tBu)OH | 1158,42 |
| 6* | -8-Amino-3,6-dioxaoctanoyl- Asp(tBu)-Ala-His(trt)-Ser(tBu)-Phe-Ser(tBu)OH | 1218,47 |
| 7* | 8-Amino-3,6-dioxaoctanoyl-Leu-Ile-Lys(tfa)-Lys(tfa)-Pro-Phe-OH | 1082,16 |

| | | |
|---|---|---|
| * à titre de comparaison | | |

### Couplage des peptides protégés:

### Etape 1 : Couplage des peptide N° 1, 2, 3, 4, 5, 6 ou sur le dérivé squarate

Le composé obtenu à l'étape de l'exemple 3 (100 mg, 1,35 10⁻⁴ mole) est dissous dans 15 ml de solution aqueuse de Na₂CO₃ pH 9,4. Le peptide protégé (1,6 10⁻⁴ mole) est introduit en maintenant le pH à 9,4 par ajout de Na₂CO₃. Si le peptide n'est pas soluble dans l'eau, quelques gouttes de DMF sont ajoutées jusqu'à dissolution complète. Après 48h de réaction à température ambiante, le milieu est précipité dans un mélange éthanol/éther éthylique. Le précipité est filtré puis séché.

### Etape 2 : déprotection

Le composé obtenu à l'étape 1 ci-dessus est dissout dans un mélange de 10 cm³ de TFA/TIS/H₂O dans les proportions 90/5/5. Le milieu est agité 5h à température ambiante puis le solvant est évaporé sous pression réduite. Le résidu est repris dans l'éther éthylique et le précipité est filtré puis séché. Le produit est ensuite purifié par HPLC préparative sur une colonne Symmetry® avec un éluant constitué d'eau/TFA PH 3/ CH₃CN.

### Couplage d'un peptide protégé TFA peptide n°7 :

2.10 mg du composé obtenu à l'étape 5 de l'exemple 3 sont dissous dans 20 ml d'eau et le pH est ajusté à 9 avec une solution de NaCO₃. 350 mg de peptide protégé (peptide N°7) sont ajoutés et le pH est ajusté à 9,2. Le milieu est agité à température ambiante puis la solution est dialysée sur une membranes de seuil de coupure 1 kD pendant 48h puis chromatographiée sur colonne RP-18 (mélange MeOH/eau (50/50)).

| N° | Structure | PM | Spectrométrie de masse |
|---|---|---|---|
| 1 * | 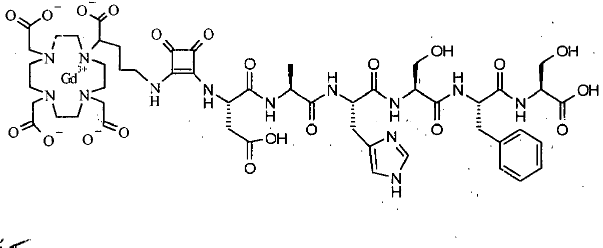 | 1355,43 | ES-m/z: 1354,2 |
| 2* | 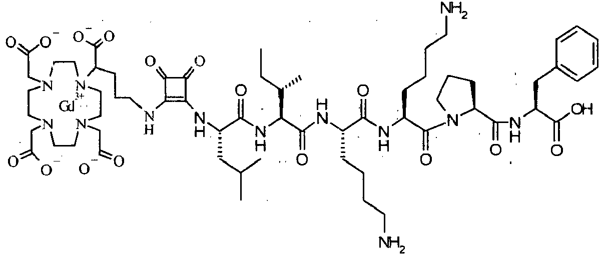 | 1437,75 | ES-m/z : 1436,3 |
| 3 | 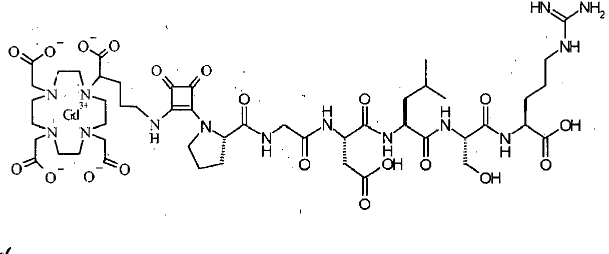 | 1336,47 | ES-m/z : 1335,1 |
| 4* | 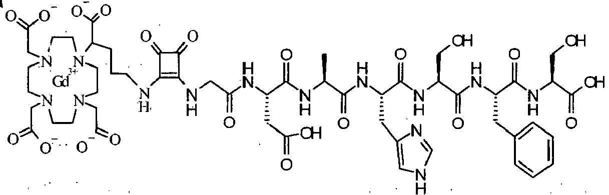 | 1412,48 | ES-m/z : 1412 |
| 5* | 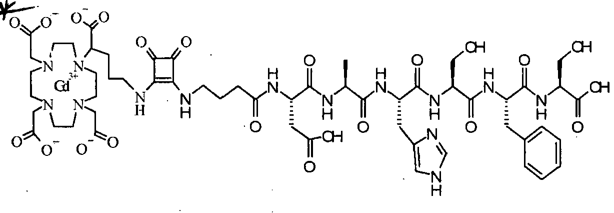 | 1440,53 | ES-m/z : 1439 |
| 6* | 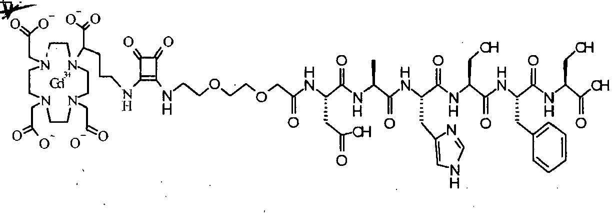 | 1500,59 | ES-m/z : 1448 |
| 7* | 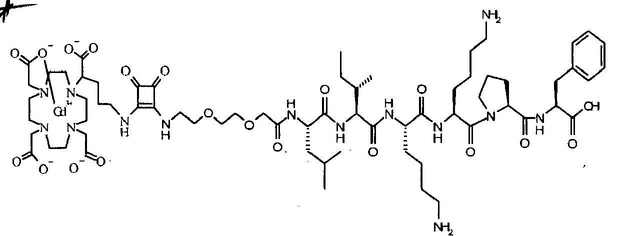 | 1582,91 | ES-m/z : 1582 |

| | | | |
|---|---|---|---|
| * à titre de comparaison | | | |

### Exemple 5 :Couplages de chélates dérivés du DOTA et du PCTA

### DOTA carboxylate :

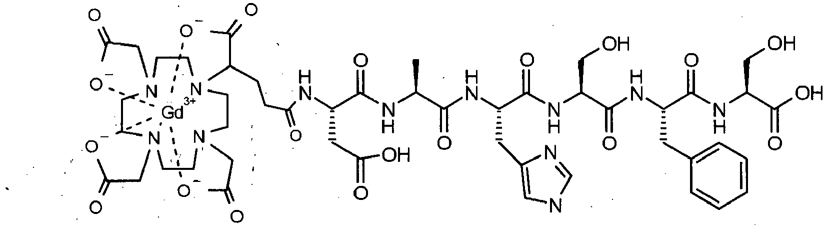

Couplage peptidique d'un DOTA carboxylate sur un peptide protégé en présence d'EDCI et déprotection dans le TFA.
S M : ES-, M/Z=1274 avec z=1

### DOTA thio-urée :

Condensation en milieu basique sur un isothiocyanate selon l'exemple 2 et déprotection dans le TFA.

| N° | Structure | PM | Spectrométrie de masse |
|---|---|---|---|
| 1* | 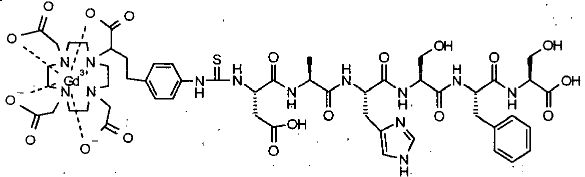 | 381,53 | ES-m/z: 1379,5 |
| 3 | 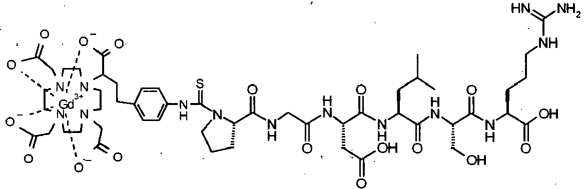 | 362,57 | ES-m/z : 1361 |

| | | | |
|---|---|---|---|
| * à titre de comparaison | | | |

### PCTA carboxylate :

### Couplage peptidique et déprotection dans le TFA.

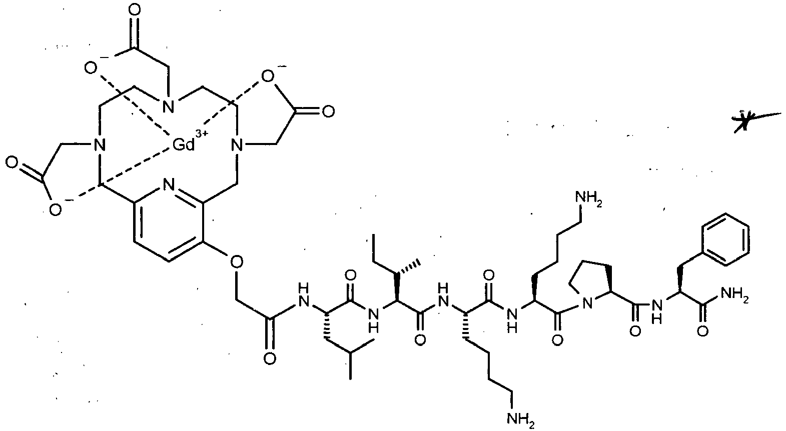

SM : ES+, M/Z= 1336 avec z=1

### PCTA thiourée:

Condensation en milieu basique sur un isothiocyanate selon l'exemple 2 et déprotection dans le TFA.

| N° | Structure | PM | Spectrométrie de masse |
|---|---|---|---|
| 1* | 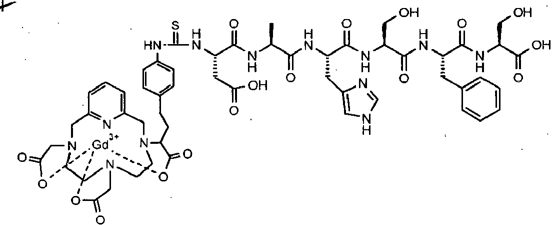 | 1358,52 | ES+m/z : 1359,8 |
| | Molecular Weight =1358,52 | | |
| 3 | 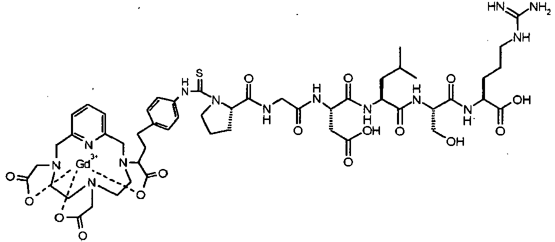 | 1339,56 | ES+m/z : 1341 |

| | | | |
|---|---|---|---|
| * à titre de comparaison | | | |

### PCTA squarate :

Selon l'exemple 4 étapes 1 et 2.

| N° | Structure | PM | Spectrométrie de masse |
|---|---|---|---|
| 1* | 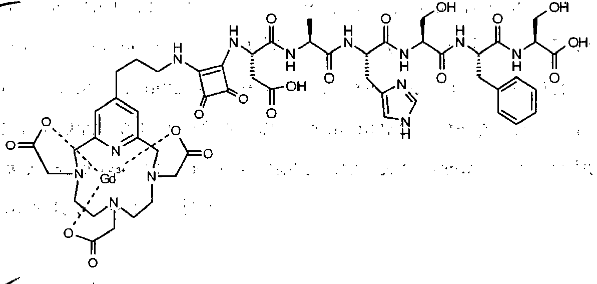 | 1332,42 | ES+m/z 1333,6 |
| 2* | 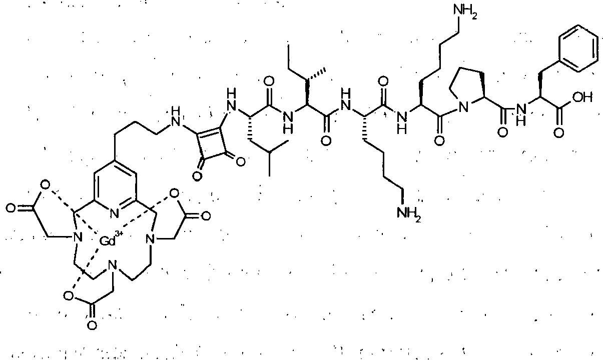 | 1414,74 | ES+ m/z : 1416 |
| 3 | 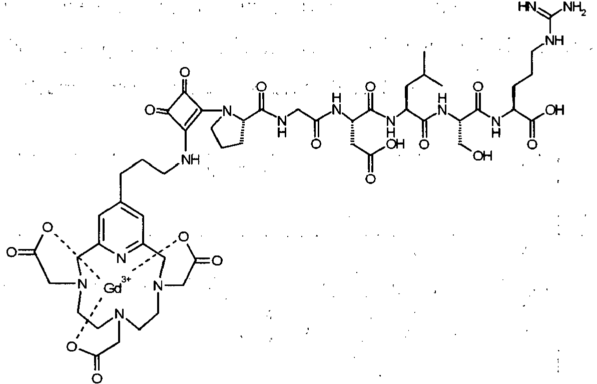 | 1313,46 | ES+m/z : 1314 |

| | | | |
|---|---|---|---|
| * à titre de comparaison | | | |

### Exemple 6: Préparation de lipopeptides

### Etape 1 : Hexadécanoate de pentafluorophényle

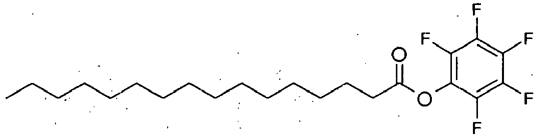

A une solution constituée de 3,85 g d'acide hexadécanoique et de 2,76 g de pentafluorophénol dans 20 ml de dioxane et 8ml de DMF, sont ajoutés à 0°C, 3,1g de dicyclohexylcarbodiimide (DCC) dans 20 ml de dioxane. Le mélange est agité une nuit à température ambiante puis le milieu réactionnel est filtré et la solution obtenue est évaporée sous pression réduite. L'huile ainsi récupérée est reprise dans le cyclohexane pour donner un solide blanc (5 g).
Température de fusion: 42-44°C.

### Etape 2 : Acylation des peptides

Les peptides sont synthétisés sur phase solide en utilisant 2,5 équivalents de chaque aminoacide protégé Fmoc à chaque cycle de couplage. L'activation des acides carboxyliques est réalisée avec HATU, N-méthylmorpholine dans le DMF. Les groupements Fmoc sont coupés par traitement à la pipéridine (20% dans le DMF). Après introduction du dernier aminoacide de la séquence peptidique et coupure du groupement protecteur Fmoc, l'amine N-terminale du peptide est acylé par le composé préparé à l'étape 1 (2,5 équivalents) dissous dans CH₂Cl₂ en présence de HoBt et de N-méthylmorpholine. Le peptide est ensuite libéré de la résine et les groupements protecteurs des fonctions latérales sont coupés par action d'un mélange acide trifluoroacétique/thioanisole (95/5) durant 30 minutes à 0°C puis 2h à température ambiante. La résine est éliminée et le solvant est évaporé sous pression réduite. Le lipopeptide est précipité dans l'éther éthylique. Les produits sont purifiés par HPLC préparative sur colonne Vydac ODS® en éluant avec un mélange Eau/acétonitrile/TFA.

| Structure | Poids moléculaire | ES+ m/z |
|---|---|---|
| 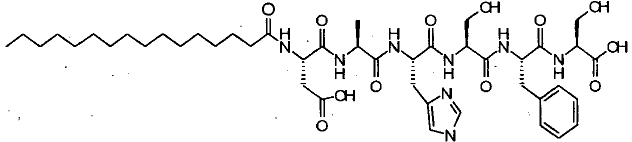 | * 901,08 | 902,5 |
| 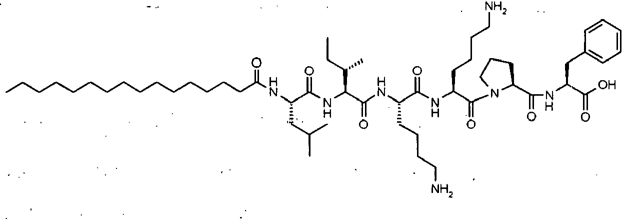 | * 983,40 | 986 |
| 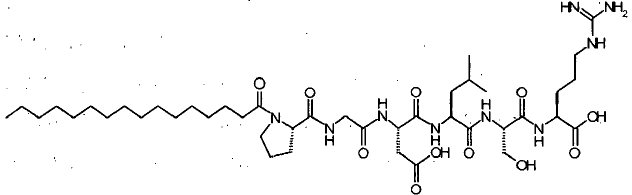 . | 882,12 | 884 |

| | | |
|---|---|---|
| * à titre de comparaison | | |

### EXEMPLES PARTIE II : mise en évidence de l'efficacité de PEPTIDES P

### Exemple II.1 : Peptide LIKKPF (SEO ID No 11) *

L'affinité du peptide LIKKPF (SEQ ID No 11) est mesurée sur PS immobilisée (plaque ELISA) Le peptide est incubé à des concentrations croissantes de 5.10⁻¹⁴ M à 4,2.10⁻⁷ M. L'annexin V-biotine est alors ajoutée à la concentration de 5.10⁻¹⁰ M qui correspond à sa valeur K_{d} pour la PS. L'incubation à 37°C se prolonge pendant 1.5 heure. L'annexine V liée est détectée par ajout d'une solution de streptavidine-HRP, puis d'une solution de substrat ABTS contenant 0.05% d'H₂O₂. La DO₄₀₅ est mesurée pour calcul de la valeur IC₅₀ du peptide.

### Exemple II.2. Produit de contraste USPIO-LIKKPF *

L'affinité de l'USPIO-LIKKPF est mesurée sur PS immobilisée (120 µg/mL). Après saturation des sites non spécifiques, l'USPIO-LIKKPF est incubé à des concentrations croissantes (122 nM - 4 mM en fer) pendant 2 heures à 37°C. Après lavage des puits, les molécules liées à la PS sont digérées dans un volume de HCl 5 N. Les échantillons sont digérés dans un volume de HCl 5N, 48h à 37°C, et le dosage de fer est fait par Bleu de Prusse (DO₆₃₀).

L'IRM vitro est réalisée sur des cellules apoptotiques (ou contrôles) pré-incubées avec l'USPIO-LIKKPF ou l'USPIO non fonctionnalisé (3 ou 4 mM en fer) pendant 2 heures à 37°C. L'apoptose des cellules Jurkat est induite par traitement à la camptothécine 2 µM pendant 24 heures. Après incubation avec les USPIO, les cellules marquées sont transférées dans une solution de gélatine 0,2% pour l'analyse par IRM (Bruker AVANCE-200, 4,7 T, TR/TE = 3000/20 ms, 30 échos, matrice = 256 x 256, FOV = 4 cm, épaisseur de la coupe = 1 mm) dans des puits de plaque ELISA.

### Exemple II.3 : Produit de contraste Gd-DTPA-LIKKPF *

Les peptides LIKKPF (SEQ ID No 11) et FKIPKL (peptide « scramble » - séquence mélangée) sont greffés séparément sur un chélate de Gd-DTPA. Les peptides protégés par du TFA sur deux Lys sont attachés au linker 8-amino-3,6-dioxooctanoyl. Le peptide protégé est couplé au DTPA-isothiocyanate (NCS-Bz-DTPA), puis les Lys sont déprotégées dans un milieu basique. Des cellules Jurkat apoptotiques en suspension (2.10⁶ cellules/mL) sont incubées en présence du produits Gd-DTPA-LIKKPF aux concentrations 400, 200 et 100 µM en Gd. Les cellules sont incubées 2 heures à température ambiante. Après rinçage et centrifugation, le culot cellulaire est minéralisé dans des conditions acides et les quantités de Gd sont mesurées par ICP-MS (inductively coupled plasma - mass spectrometry).
L'efficacité de ciblage du produit Gd-DTPA-LIKKPF par IRM a été aussi évaluée sur un modèle de foie apoptotique chez la souris. L'apoptose est induite par une injection i.v. (veine caudale) d'anticorps monoclonal anti-Fas chez la souris Balb/c anesthésiée (Blankenberg et al., 19981, 1999 Rodriguez et al., 1996).
Avant l'acquisition IRM, les souris sont anesthésiées au pentobarbital. Les expériences sont réalisées à l'aide d'un spectromètre Bruker de 200 MHz (4,7 T) équipé d'un aimant vertical et d'un système de mini-imagerie. Les images sont acquises à l'aide d'une séquence MSME (multi-slice-multi-echo) avec les paramètres suivants : TR/TE = 307,4 /14,7 ms, matrice = 256, FOV = 5 cm, épaisseur de la coupe = 3 mm, 8 coupes axiales (qui couvrent complètement la région abdominale incluant les reins), TA = 5 minutes 14 secondes. Les images sont acquises avant et après l'injection des produits de contraste, jusqu'à 1,5 heure. L'intensité de signal (SI) est mesurée dans le foie. La valeur SI est aussi mesurée dans un tube rempli avec une solution aqueuse 2% de gélatine enrichie avec 50 µM Gd-DTPA, utilisé comme produit de référence, dans une région en dehors de l'image de la souris qui correspond au bruit de fond. Le protocole d'IRM est le suivant : (1) l'IRM pré-contraste commence 1h15 après l'injection d'anti-Fas ; (2) le produit de contraste est injecté dans la veine fémorale à la dose 60 µmol Gd/kg environ 1h45 après l'injection d'anti-Fas ; (3) plusieurs acquisitions post-contraste sont réalisées pendant 1h30. Un protocole de compétition est réalisé par injection de 100 µmol peptide/kg 10 minutes avant l'injection du produit de contraste Gd-DTPA-LIKKPF.

L'efficacité de ciblage du produit Gd-DTPA-LIKKPF a été aussi évaluée sur un autre modèle d'apoptose : la souris apoE^{-/-}, modèle d'athérosclérose.⁻ Des souris femelles C57B1 ApoE^{-/-} âgées environ de 15 mois sont soumises à un régime riche en cholestérol pendant 3 mois avant les études IRM Pour l'acquisition par IRM les animaux sont anesthésiés au pentobarbital. L'agent de contraste Gd-DTPA-LIKKPF est injecté par voie i.v. à la dose de 100 µmol Gd/kg. Toutes les.images sont acquises au niveau de l'aorte abdominale, en particulier la région proche du rein qui est connue pour le développement de plaques d'athérome en raison de la présence des branches artérielles.
Les paramètres de la séquence RARE (Rapid Acquisition with Relaxation Enhancement) sont ajustés en utilisant un tube référence rempli d'une solution 1 mM de Gd-DTPA. Les paramètres d'acquisition d'images sont les suivants : TR = 470,9 - 1048,5 ms, TE = 4 ms, facteur RARE = 1 - 4, NEX = 4, matrice = 256 x 256, FOV = 2,3 cm, largeur-spectrale = 33,33 kHz, épaisseur de la coupe = 0,8 mm, résolution spatiale = 90 µm.

Pour les deux modéles, les valeurs SI sont mesurées dans différentes régions d'intérêt (au niveau de la paroi artérielle de l'aorte abdominale ou foie entier) à l'aide du logiciel d'analyse d'image OSIRIS. Les régions sont d'abord dessinées sur les images post-contraste, puis dupliquées sur les images pré-contraste. La valeur SI est mesurée sur toutes les coupes d'images où la paroi artérielle et le foie sont visibles. Finalement, les valeurs SI obtenues pour des coupes sériées d'aorte sur une longueur de 3,2 - 8 mm sont moyennées pour chaque animal. Le ΔSNR% est calculé selon l'équation suivante :
ΔSNR% = [(SIₚₒₛₜ / SD_{bruit}) - (SI_{prè} / SD_{bruit})]/[SI_{pré} / SD_{bruitt}] X 100 où SD_{bruit} est la déviation standard du bruit mesuré sur une région en dehors de l'animal.

### II.4 résultats : affinité des PEPTIDES P

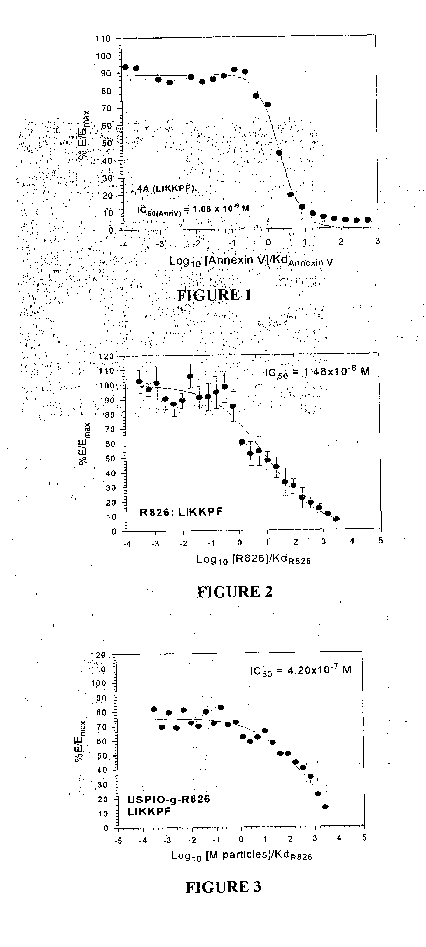

Le demandeur a identifié plusieurs séquences consensus :
- VLGERG (SEQ ID No 5) à titre de comparaison et LIKKPF (SEQ ID No 11) à titre de comparaison
- D-A-H-S-X7-S (SEQ ID No 2) à titre de comparaison, oú X7 peut être F ou L
- P-G-D-L-X8-X9 (SEQ ID No 3), où X8 est choisi parmi S ou V et X9 est choisi parmi T ou R
H-G-X10-L-S-X11 (SEQ ID No 4) à titre de comparaison et ses équivalents fonctionnels, où avantageusement X10 est choisi parmi D ou H, et X11 est choisi parmi T ou 1
L'affinité (en ordre de grandeur) de ces peptides pour la PS (constante de dissociation apparente K*_{d}) est comprise entre 10⁻⁶ et 10⁻⁹ M.
Le peptide LIKKPF (SEQ ID No 11) à titre de comparaison à forte spécificité a été particulièrement bien caractérisé.
La spécificité d'interaction du peptide avec la PS est confirmée à l'aide du test de compétition à l'annexine V. La valeur IC₅₀ de l'annexine V est égale à 1,08.10⁻⁹ M (figure 1).

### Exemple II.5 Peptide LIKKPF (SEQ ID No 11) à titre de comparaison

Le test vitro sur PS immobilisée permet de calculer une valeur IC₅₀ (figure 2).

### Exemple II.6. Produit de contraste USPIO-PEG-PEPTIDE P avec PEPTIDE P étant LIKKPF (SEQ ID No 11) (également désigné USPIO-LIKKPF) à titre de comparaison

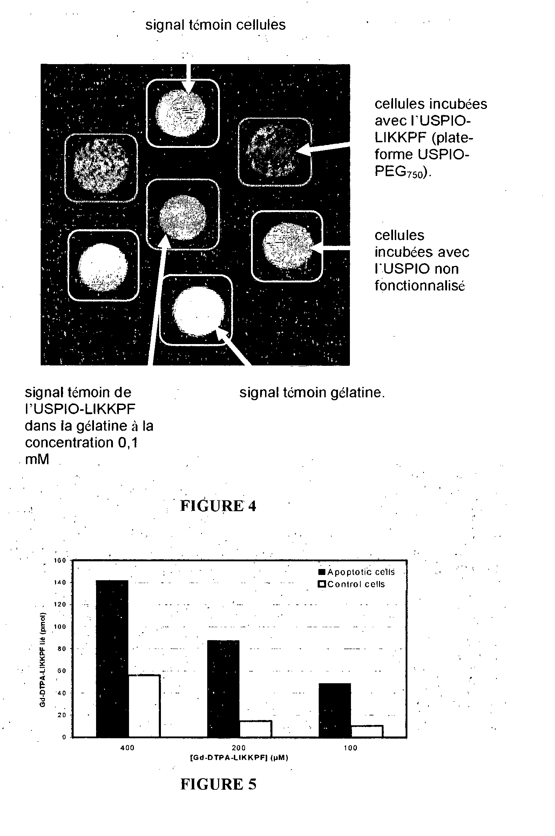

Le test d'interaction de l'USPIO-LIKKPF avec la PS immobilisée permet de mesurer une valeur K*_{d} de 7,85.10⁻⁸ M. La spécificité d'interaction de l'USPIO-LIKKPF avec la PS est vérifiée avec un test de compétition à l'annexine V. La valeur IC₅₀ est égale à 4.2.10⁻⁷ M. (figure 3) L'IRM sur cellules montre que USPIO-LIKKPF se lie spécifiquement aux cellules apoptotiques En effet, l'intensité de signal IRM enregistrée avec ces cellules est plus faible que celle obtenues avec les cellules contrôles. De plus, la baisse de signal IRM induite par l'incubation avec l'USPIO-LIKKPF est plus importante que celle enregistrée avec l'USPIO non fonctionnalisé (USPIO-PEG non couplé au peptide LIKKPF (SEQ ID NO 11))- cf Figure 4 (cellules apoptotiques ; la colonne gauche correspond à la concentration 3 mM, la colonne droite correspond à la concentration du Fe de 4 mM.
L'ensemble de ces résultats démontre le ciblage spécifique de l'USPIO-LIKKPF pour des cellules apoptotiques.

### Exemple II. 7 produit de contraste Gd-DTPA-LIKKPF à titre de comparaison

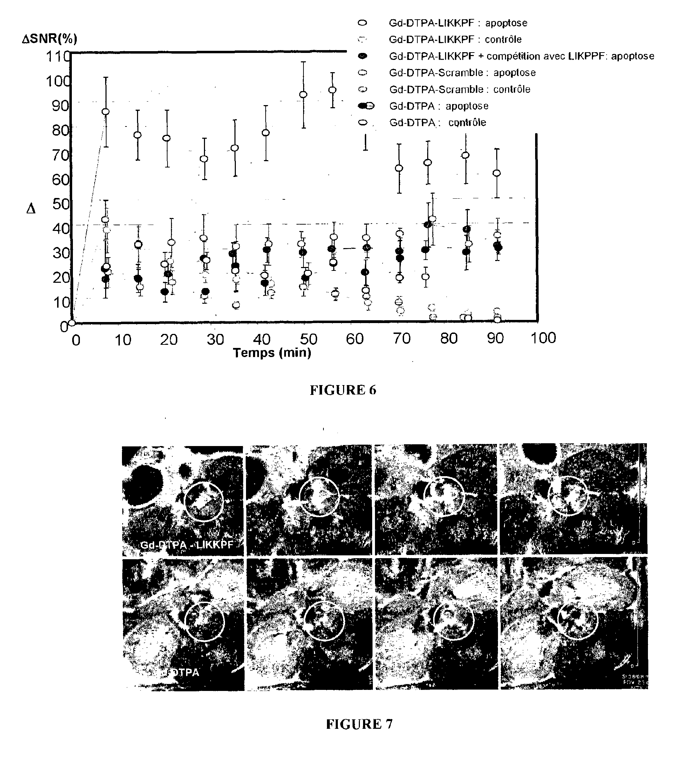

Le greffage du peptide LIKKPF (SEQ ID NO.11) sur le produit de contraste Gd-DTPA permet la conservation du ciblage des cellules apoptotiques. En effet, le dosage de ICP-MS de cellules apoptotiques incubées avec le Gd-DTPA-LIKKPF prouve que leur interaction, concentration-dépendante, est supérieure à la capture du produit par les cellules contrôles (Figure 5).
Après injection i.v. de 60 µmol Gd/kg de produit, le suivi dynamique du signaL IRM du foie apoptotique de souris (figure 6) montre un rehaussement avec le Gd-DTPA-LIKKPF supérieur à celui mesuré avec le Gd-DTPA-FKIPKL (scramble) ou le Gd-DTPA non fonctionnalisé. La pré-injection du peptide LIKKPF (SEQ ID NO 11), à la dose 100 µmol/kg, bloque le rehaussement causé par le Gd-DTPA-LIKKPF ce qui témoigne de la spécificité d'interaction entre le Gd-DTPA-LIKKPF et la PS. Par ailleurs, les trois produits (Gd-DTPA-LIKKPF, Gd-DTPA-FKIPKL et Gd-DTPA) n'ont permis de n'enregistrer qu'un rehaussement partiel de signal chez la souris saine. L'ensemble de ces données prouve que le produit de contraste Gd-DTPA-LIKKPF cible spécifiquement l'apoptose in vivo via la PS.

### Exemple II.8 : Imageric de tissu vasculaire apoptotique à titre de comparaison

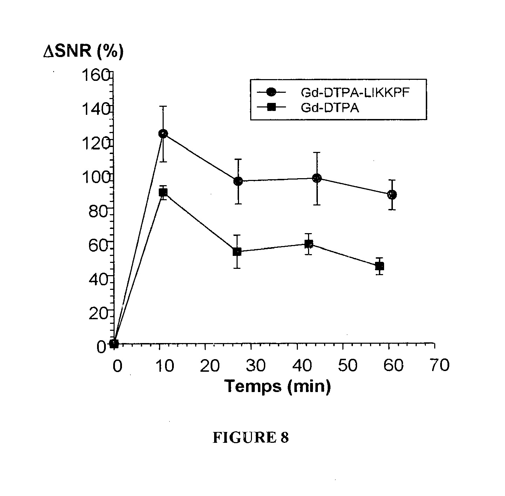

Dans un modèle d'athérosclérose chez la souris (souris apoE^{-/-}), l'injection i.v. de produit Gd-DTPA-LIKKPF. à la dose 100 µmol Gd/kg, permet de détecter de manière dynamique un rehaussement de signal supérieur à celui mesuré avec le produit non greffé (Gd-DTPA) au niveau de l'aorte abdominale (figures 7 et 8 - Séquence RARE, 27 minutes après injection de produit de contraste - IRM de coupes sériées de l'aorte abdominale sur une longueur de 3,2 mm). Ce résultat indique que le produit Gd-DTPA-LIKKPF est capable de cibler spécifiquement la plaque d'athérome, via l'interaction avec la PS.

### Exemple II.9 : Imagerie de tissus nerveux apoptotique à titre de comparaison

Dans un modèle de maladie de Parkinson, avec injection de produit USPIO- LIKKPF (produit de l'exemple 1).

Des études IRM ont été réalisées sur des souris C57BI/6 traitées au MPTP (ou son métabolite le MPP+ pour les cultures cellulaires), neurotoxine induisant un syndrome parkinsonien"

En culture cellulaire la mort des neurones dopaminergiques par apoptose a été vérifiée à partir de cerveaux de rats par immunohistochimie et détection de l'activité enzymatique de la caspase 3. Pour la détection IRM en culture cellulaire, les cellules saines et traitées au MPP+ ont été mise en contact avec des USPIO-LIKKPF. Les cellules malades montrent un signal plus sombre que les autres, démontrant le ciblage du peptide sur les cellules apoptotiques. La vitesse de relaxation normalisée et la concentration en fer montrent que les cellules malades ont capté une plus grande quantité d'agent de contraste vectorisé.
L'immunohistochimie et l'IRM sont très révélatrices. Les souris malades et saines ont été étudiées au 4è, 5è, 6è jour de traitement, et une et trois semaines après la fin du traitement au MPTP. L'imagerie montre une évolution du signal au cours du traitement, le ciblage étant plus marqué au 5è jour puis diminuant après. Les coupes réalisées avec ces USPIO non vectorisés (sans peptide) ne montrent pas de ciblage marqué. La localisation IRM des zones malades correspond aux zones contenant des neurones dopaminergiques (immunohistochimie : détection des neurones dopaminergiques par mesure de la tyrosine hydroxylase au niveau des noyaux gris centraux).

## Revendications

1. Composé de formule générale (I) suivante :
Signal - Lien - Peptide (I)
dans laquelle
Signal représente une entité signal choisie parmi :
- un chélate couplé à un métal paramagnétique M ou à un radionucléide,
- une nanoparticule superparamagnétique revêtue d'une couche organique, de préférence ayant un noyau d'oxyde de fer et
- une molécule fluorescente utilisée en imagerie optique ;
Lien, présent ou non, représente une liaison chimique et
Peptide représente un peptide comprenant un peptide ciblant l'apoptose, le peptide ciblant l'apoptose étant le peptide de formule suivante:
P-G-D-L-X8-X9 (3) (SEQ ID No 3)
dans laquelle X8 représente la sérine ou la valine et X9 représente la thréonine ou l'arginine ;
et les sels pharmaceutiquement acceptables de ces composés
à l'exception des composés de formules suivantes : 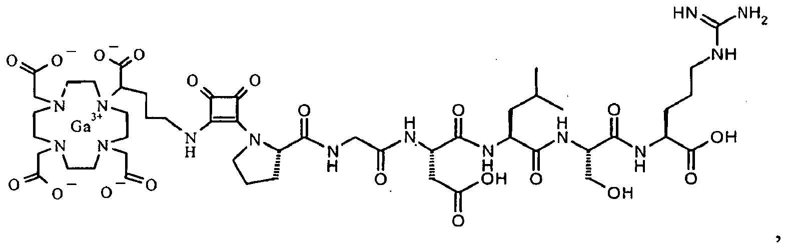 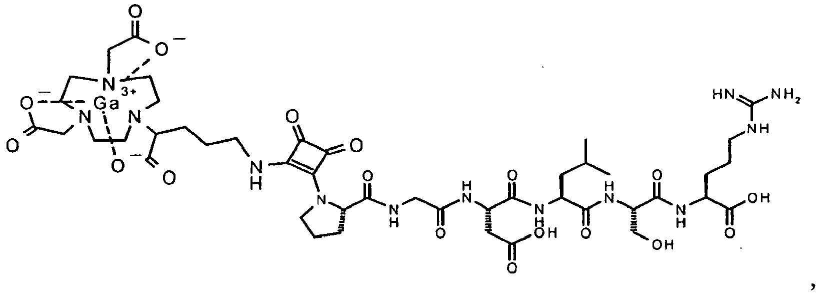 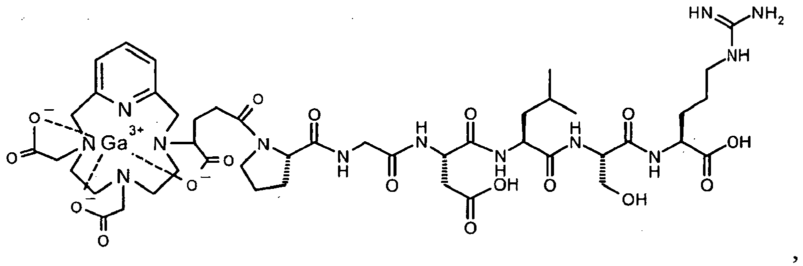 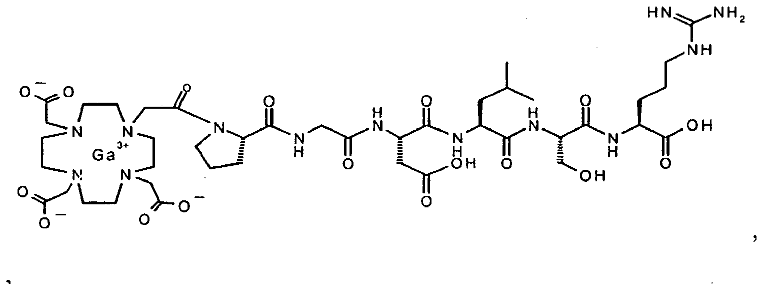 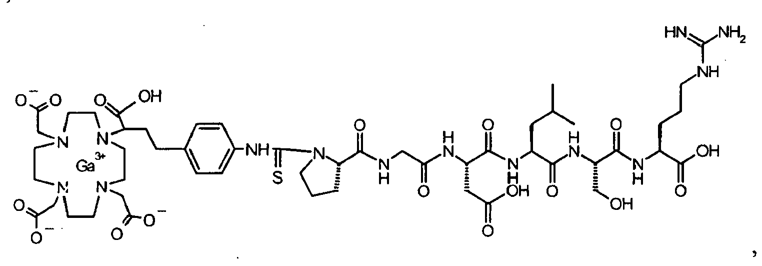 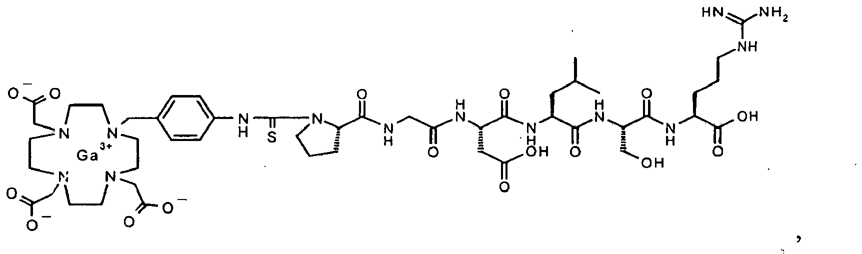 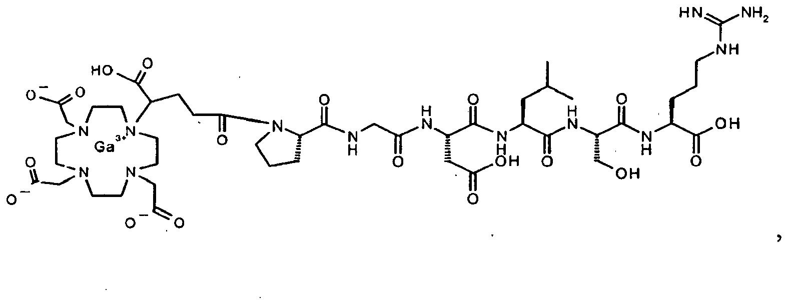 et

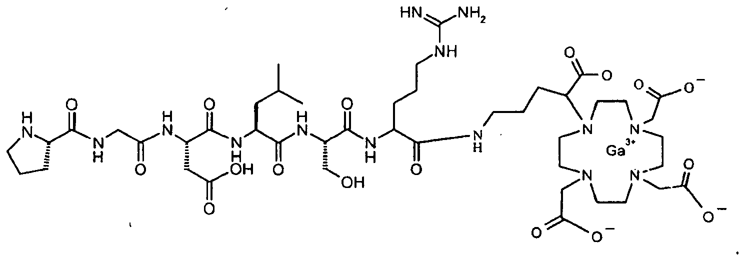

2. Composé selon la revendication 1 dans laquelle Peptide est un peptide de formule PGDLSR (SEQ ID No 3).

3. Composé selon la revendication 1 ou 2 dans laquelle Signal est un chélate couplé à un métal paramagnétique M, M étant choisi avantageusement parmi Gd, Mn, Fe, Dy et Tm.

4. Composé selon la revendication 1 ou 2 dans laquelle Signal est un chélate couplé à un radionucléide.

5. Composé selon la revendication 4, dans laquelle le radionucléide est le Ga68 pour l'imagerie PET.

6. Composé selon les revendications 3 à 5 dans laquelle le chélate est choisi parmi : DOTA, DTPA, DO3A, HPDO3A, TRITA, TETA, BOPTA, NOTA, PCTA, DOTMA, AAZTA, HOPO et leurs dérivés.

7. Composé selon la revendication 1 à 6 dans laquelle Lien représente :
a) un groupe de formule: Q1-1-Q2,
dans laquelle Q1 et Q2 identiques ou différents représentent O, S, NH, CO₂, -NHCO, CONH, NHCONH, NHCSNH, SO₂NH- ou NHSO₂-,
et 1 représente un groupe alkyle, alcoxyalkyle, polyalkoxyalkylène, alcényle, alcynyle, alkyle interrompu par un ou plusieurs squarate, par un ou plusieurs aryles, avantageusement phényle, ou par un ou plusieurs groupes choisis parmi -NH-, -O-, -CO-, -NH(CO)-, -(CO)NH-,-O(CO)-, ou -(OC)O- ;,
b) un groupe (CH₂)ₙ, (CH₂)ₙ-CO-, -(CH₂)ₙNH-CO- avec n = 2 à 10 , (CH₂CH₂O)_{q}(CH₂)ᵣCO- , (CH₂CH₂O)_{q}(CH₂)ᵣ-NH-CO- avec q = 1-10 et r=2-10, (CH₂)ₙ-CONH - , (CH₂)ₙ-CONH-PEG, (CH₂)ₙ-NH-, 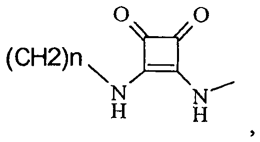 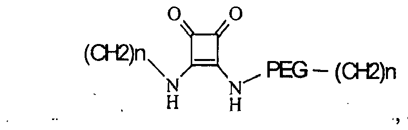 avec n=1 à 5 et avantageusement n=4 ou 5, HOOC-CH₂-O-(CH₂)₂-O-(CH₂)₂-O-CH₂-COOH ; HOOC-(CH₂)₂-CO₂-(CH₂)₂-OCO-(CH₂)₂-COOH ; HOOC-CH(OH)-CH(OH)-COOH; HOOC-(CH₂)ₙ-COOH; NH₂-(CH₂)ₙ-NH₂, avec n=1-20; NH₂-(CH₂)ₙ-CO₂H ; ou NH₂-CH₂-(CH₂-O-CH₂)ₙ-CO₂H avec n=1 à 10.

8. Composé selon la revendication 1 ou 2 dans laquelle Signal représente une nanoparticule superparamagnétique revêtue d'une couche organique, de préférence ayant un noyau en oxyde de fer.

9. Composé selon la revendication 8 dans laquelle la couche organique est une couche gem-bisphosphonate.

10. Composé selon la revendication 1 ou 2 dans laquelle Signal représente une nanoparticule lipidique comprenant au moins un chélate, avantageusement choisi parmi : DOTA, DTPA, DO3A, HPDO3A, TRITA, TETA, BOPTA, NOTA, PCTA, DOTMA, AAZTA, HOPO et leurs dérivés.

11. Composition pour imagerie médicale comprenant au moins un composé de formule générale (I) selon l'une des revendications 1 à 10 et un excipient pharmaceutiquement acceptable, avantageusement destinée à l'administration par voie parentérale.

12. Procédé de préparation de la composition selon la revendication 11 comprenant l'addition d'un composé de formule générale (I) selon l'une des revendications 1 à 10 à un milieu injectable comprenant l'excipient pharmaceutiquement acceptable .

13. Composé de formule générale (I) selon les revendications 1 à 10, ou composé de formule (I) Signal-Lien-Peptide dans laquelle Lien et Peptide sont tels que définis dans les revendications 1 à 9 et Signal représente une nanoparticule lipidique, ladite nanoparticule étant porteuse d'au moins un chélate capable d'être couplé à un métal paramagnétique, pour son utilisation en tant qu'agent de diagnostique de maladies associées à l'apoptose.

14. Utilisation d'un composé (I) selon l'une des revendications 1 à 10, ou composé de formule (I) Signal-Lien-Peptide dans laquelle Lien et Peptide sont tels que définis dans les revendications 1 à 9 et Signal représente une nanoparticule lipidique, ladite nanoparticule étant porteuse d'au moins un chélate capable d'être couplé à un métal paramagnétique, pour la préparation d'une composition de diagnostique de maladies associées à l'apoptose, avantageusement choisies parmi les maladies neurodégénératives chroniques, les maladies ischémiques et les pathologies inflammatoires.

15. Utilisation d'un peptide ciblant l'apoptose de formule définie dans la revendication 1, pour la préparation d'une composition de diagnostique de maladies associées à l'apoptose.

## Patentansprüche

1. Verbindung der folgenden allgemeinen Formel (I):
Signal-Linker-Peptid (I)
worin:
Signal eine Signaleinheit ausgewählt aus den Folgenden bedeutet:
- einem Chelat, das an ein paramagnetisches Metall M oder an ein Radionukleid gekoppelt ist,
- einem superparamagnetischen Nanopartikel, das von einer organischen Schicht umhüllt ist, vorzugweise mit einem Eisenoxidkern, und
- einem fluoreszierenden Molekül, das in der optischen Bildgebung eingesetzt wird;
Linker, der gegebenenfalls vorhanden sein kann, bedeutet eine chemische Bindung und
Peptid bedeutet ein Peptid umfassend ein Peptid, das auf Apoptose zielsteuert, wobei es sich bei dem Peptid,
das auf Apoptose zielsteuert, um das Peptid der folgenden Formel handelt:
P-G-D-L-X8-X9 (3) (SEQ ID No 3)
worin X8 Serin oder Valin bedeutet und X9 Threonin oder Arginin bedeutet;
und pharmazeutisch unbedenkliche Salze dieser Verbindungen,
mit Ausnahme von Verbindungen der folgenden Formeln: 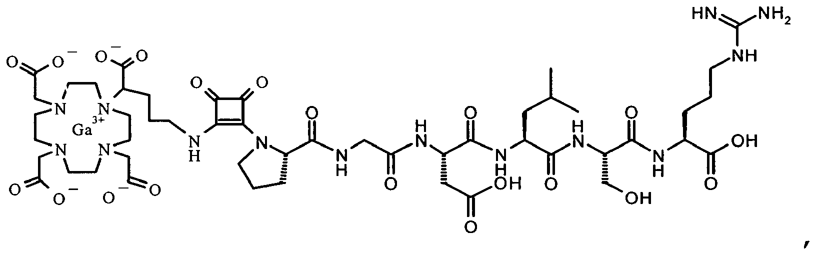 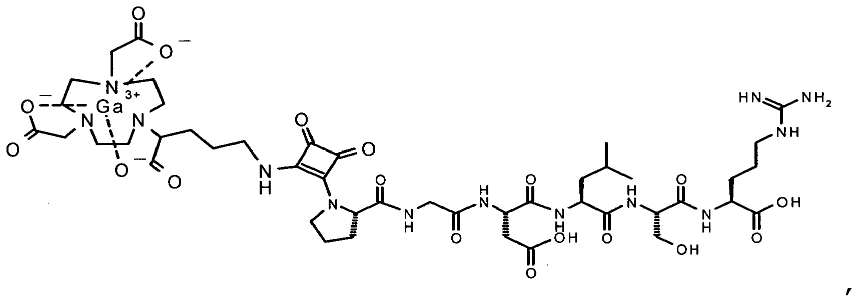 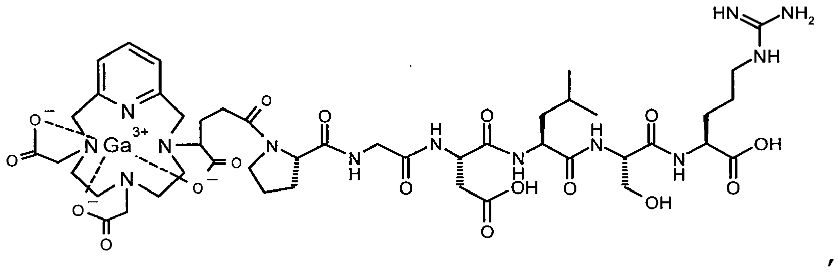 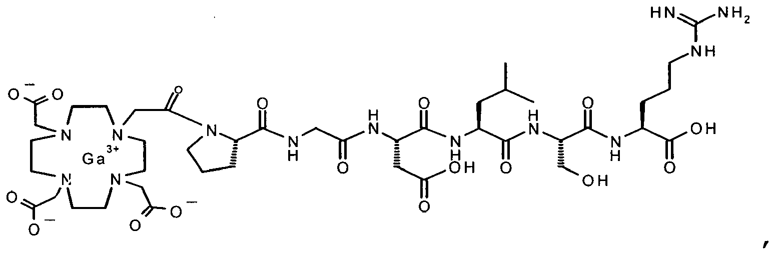 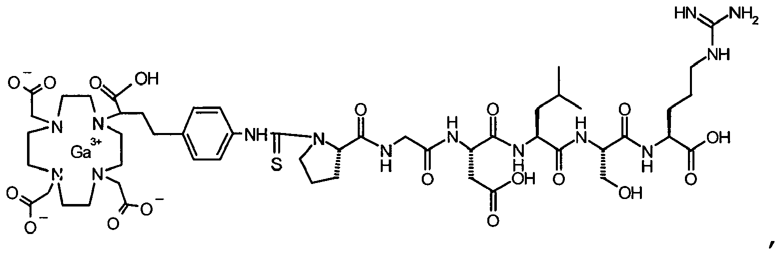 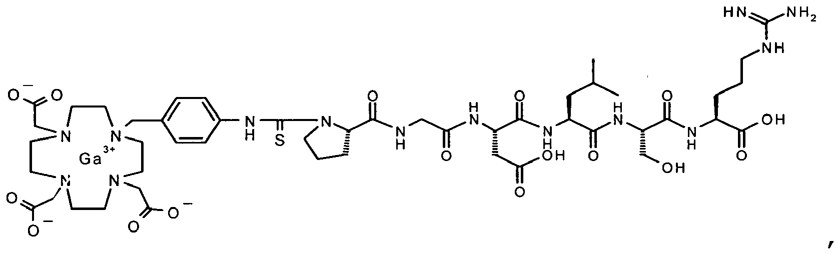 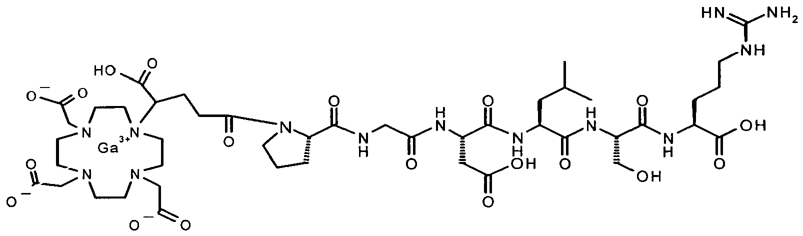 und

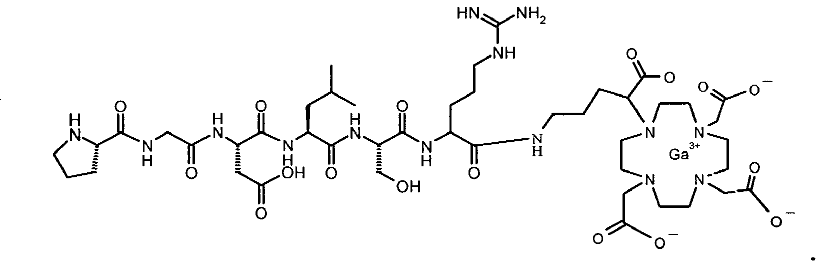

2. Verbindung nach Anspruch 1, in der es sich bei Peptid um ein Peptid der Formel PGDLSR (SEQ ID No 3) handelt.

3. Verbindung nach Anspruch 1 oder 2, in der es sich bei Signal um ein Chelat, das an ein paramagnetisches Metall M gekoppelt ist, handelt, wobei M vorzugsweise aus Gd, Mn, Fe, Dy und Tm ausgewählt ist.

4. Verbindung nach Anspruch 1 oder 2, in der es sich bei Signal um ein Chelat, das an ein Radionukleid gekoppelt ist, handelt.

5. Verbindung nach Anspruch 4, in der es sich bei dem Radionukleid um Ga68 für die PET-Bildgebung handelt.

6. Verbindung nach den Ansprüchen 3 bis 5, in der das Chelat aus den Folgenden ausgewählt ist: DOTA, DTPA, DO3A, HPDO3A, TRITA, TETA, BOPTA, NOTA, PCTA, DOTMA, AAZTA, HOPO und ihren Derivaten.

7. Verbindung nach Anspruch 1 bis 6, in der Linker Folgendes bedeutet:
a) eine Gruppe der Formel: Q1-1-Q2,
worin Q1 und Q2 gleich oder verschieden sind und O, S, NH, CO₂, -NHCO, CONH, NHCONH, NHCSNH, SO₂NH- oder NHSO₂- bedeuten,
und 1 eine Alkylgruppe, Alkoxyalkylgruppe, Polyalkoxyalkylengruppe, Alkenylgruppe, Alkinylgruppe, Alkylgruppe, die von einem oder mehreren Squaraten, von ein oder mehreren Arylen, vorteilhafterweise Phenyl, oder von einer oder mehrere Gruppen, ausgewählt aus -NH-, -O-, -CO-,-NH(CO)-, -(CO)NH-, -O(CO)-, oder -(OC)O-, unterbrochen ist, bedeutet;
b) eine Gruppe (CH₂)ₙ, (CH₂)ₙ-CO-, -(CH₂)ₙNH-CO- mit n = 2 bis 10, (CH₂CH₂O)_{q}(CH₂)ᵣ-CO-, (CH₂CH₂O)_{q}(CH₂)ᵣ-NH-CO- mit q = 1-10 und r=2-10, (CH₂)ₙ-CONH-, (CH₂)ₙ-CONH-PEG, (CH₂)ₙ-NH-, 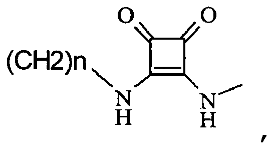 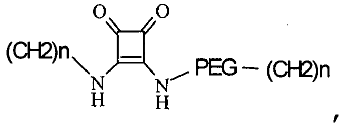 mit n=1 bis 5 und vorteilhafterweise n=4 oder 5, HOOC-CH₂-O-(CH₂)₂-O-(CH₂)₂-O-CH₂-COOH; HOOC- (CH₂)₂-CO₂- (CH₂)₂-OCO- (CH₂)₂-COOH; HOOC-CH(OH)-CH(OH)-COOH; HOOC-(CH₂)ₙ-COOH; NH₂-(CH₂)ₙ-NH₂, mit n=1-20; NH₂-(CH₂)ₙ-CO₂H; oder NH₂-CH₂-(CH₂-O-CH₂)ₙ-CO₂H mit n=1 bis 10.

8. Verbindung nach Anspruch 1 oder 2, in der Signal ein superparamagnetisches Nanopartikel, das von einer organischen Schicht umhüllt ist und vorzugweise einen Eisenoxidkern aufweist, bedeutet.

9. Verbindung nach Anspruch 8, in der es sich bei der organischen Schicht um eine gem-Bisphosphonatschicht handelt.

10. Verbindung nach Anspruch 1 oder 2, in der Signal ein Lipidnanopartikel umfassend mindestens ein Chelat, vorteilhafterweise ausgewählt aus: DOTA, DTPA, DO3A, HPDO3A, TRITA, TETA, BOPTA, NOTA, PCTA, DOTMA, AAZTA, HOPO und ihren Derivaten, bedeutet.

11. Zusammensetzung für die medizinische Bildgebung, umfassend mindestens eine Verbindung der allgemeinen Formel (I) nach einem der Ansprüche 1 bis 10 und einen pharmazeutisch unbedenklichen Grundstoff, der vorteilhafterweise für die parenterale Verabreichung bestimmt ist.

12. Verfahren zur Herstellung der Zusammensetzung nach Anspruch 11, bei dem man eine Verbindung der allgemeinen Formel (I) nach einem der Ansprüche 1 bis 10 zu einem injizierbaren Medium, das den pharmazeutisch unbedenklichen Grundstoff umfasst, hinzufügt.

13. Verbindung der allgemeinen Formel (I) nach den Ansprüchen 1 bis 10 oder Verbindung der Formel (I) Signal-Linker-Peptid, in der Linker und Peptid wie in den Ansprüchen 1 bis 9 definiert sind und Signal ein Lipidnanopartikel bedeutet, wobei das Nanopartikel mindestens ein Chelat, das mit einem paramagnetischen Metall gekoppelt werden kann, trägt, zum Einsatz als Diagnostikum für mit Apoptose assoziierten Krankheiten.

14. Verwendung einer Verbindung (I) nach einem der Ansprüche 1 bis 10 oder einer Verbindung der Formel (I) Signal-Linker-Peptid, in der Linker und Peptid wie in den Ansprüchen 1 bis 9 definiert sind und Signal ein Lipidnanopartikel bedeutet, wobei das Nanopartikel mindestens ein Chelat, das mit einem paramagnetischen Metall gekoppelt werden kann, trägt, zur Herstellung einer diagnostischen Zusammensetzung für mit Apoptose assoziierte Krankheiten, vorteilhafterweise ausgewählt aus den chronischen neurodegenerativen Krankheiten, ischämischen Krankheiten und entzündlichen Pathologien.

15. Verwendung eines Peptids, das auf Apoptose zielsteuert, mit der in Anspruch 1 definierten Formel zur Herstellung einer diagnostischen Zusammensetzung für mit Apoptose assoziierte Krankheiten.

## Claims

1. Compound of general formula (I) below:
Signal - Linker - Peptide (I)
in which:
Signal represents a signal entity chosen from:
- a chelate coupled to a paramagnetic metal M or to a radionuclide,
- a superparamagnetic nanoparticle coated with an organic layer, preferably having an iron oxide core,
and
- a fluorescent molecule used in optical imaging;
Linker, which may or may not be present, represents a chemical bond, and
Peptide represents a peptide comprising an apoptosis-targeting peptide, the apoptosis-targeting peptide being the peptide having the formula below: P-G-D-L-X8-X9 (3) (SEQ ID No 3)
in which X8 represents serine or valine and X9 represents threonine or arginine;
and the pharmaceutically acceptable salts of these compounds,
with the exception of the compounds of the formulae below: 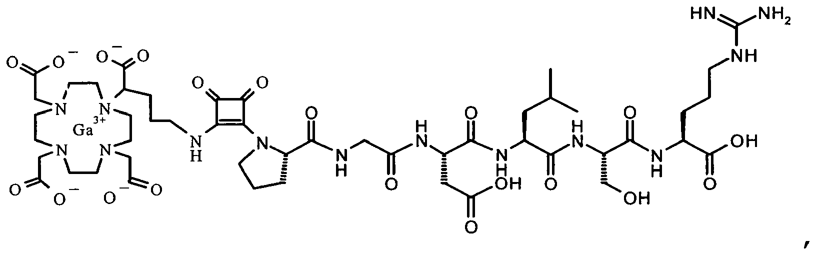 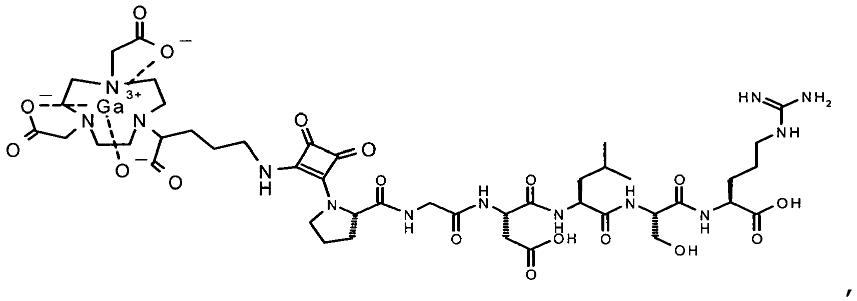 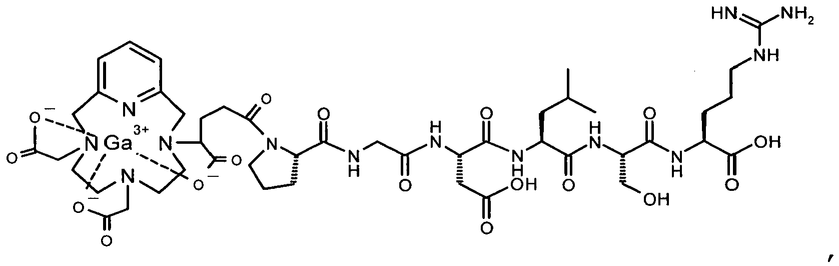 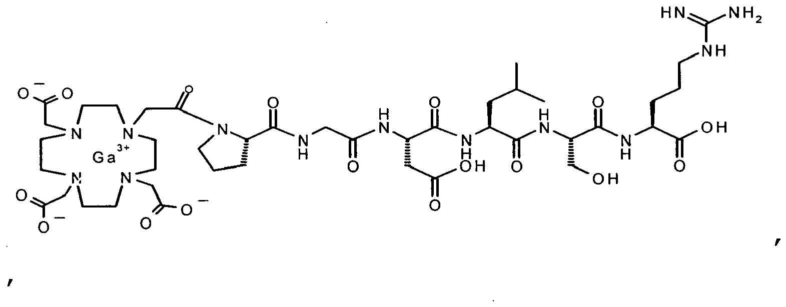 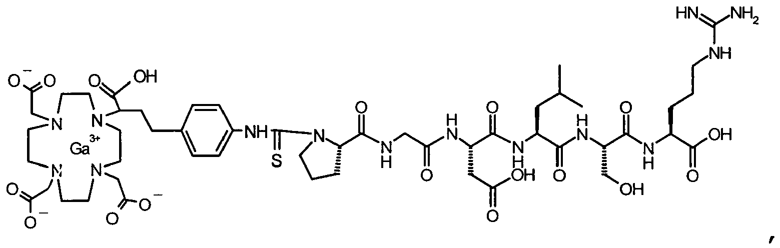 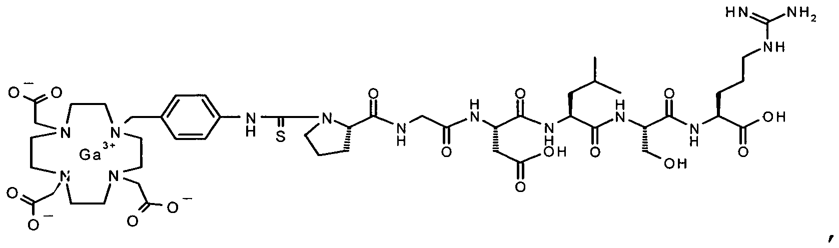 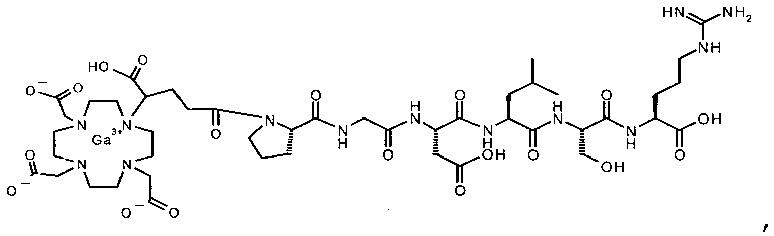 and

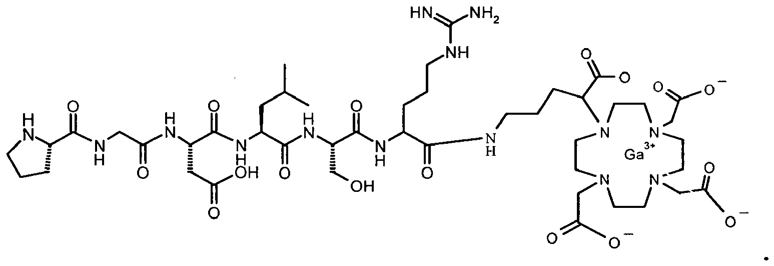

2. Compound according to Claim 1, in which Peptide is a peptide of formula PGDLSR (SEQ ID No 3).

3. Compound according to Claim 1 or 2, in which Signal is a chelate coupled to a paramagnetic metal M, M being chosen from Gd, Mn, Fe, Dy and Tm.

4. Compound according to Claim 1 or 2, in which Signal is a chelate coupled to a radionuclide.

5. Compound according to Claim 4, in which the radionuclide is Ga68 for PET imaging.

6. Compound according to Claims 3 to 5, in which the chelate is chosen from: DOTA, DTPA, DO3A, HPDO3A, TRITA, TETA, BOPTA, NOTA, PCTA, DOTMA, AAZTA, HOPO and their derivatives.

7. Compound according to Claims 1 to 6, in which Linker represents:
a) a group of formula: Q1-1-Q2,
in which Q1 and Q2, which may be identical or different, represent O, S, NH, CO₂, -NHCO, CONH, NHCONH, NHCSNH, SO₂NH- or NHSO₂-,
and 1 represents an alkyl group, alkoxyalkyl group, polyalkoxyalkylene group, alkenyl group, alkynyl group, alkyl group interrupted with one or more squarates, with one or more aryls, advantageously phenyl, or with one or more groups chosen from -NH-, -O-, -CO-,-NH(CO)-, -(CO)NH-, -O(CO)-, or -(OC)O-;
b) a (CH₂)ₙ, (CH₂)ₙ-CO-, -(CH₂)ₙNH-CO-, with n = 2 to 10, (CH₂CH₂O)_{q}(CH₂)ᵣ-CO-, (CH₂CH₂O)q(CH₂)ᵣ-NH-CO-, with q = 1-10 and r=2-10, (CH₂)n-CONH-, (CH₂)ₙ-CONH-PEG, (CH₂)ₙ-NH-, 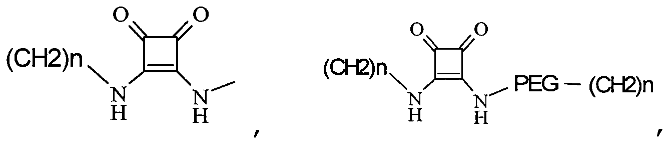 with n=1 to 5 and advantageously n=4 or 5, HOOC-CH₂-O-(CH₂)₂-O- (CH₂)₂-O-CH₂-COOH; HOOC- (CH₂)₂-CO₂-(CH₂)₂-OCO-(CH₂)₂-COOH; HOOC-CH(OH)-CH(OH)-COOH; HOOC-(CH₂)ₙ-COOH; NH₂-(CH₂)ₙ-NH₂, with n=1-20; NH₂-(CH₂)ₙ-CO₂H; or NH₂-CH₂-(CH₂-O-CH₂)ₙ-CO₂H, with n=1 to 10, group.

8. Compound according to Claim 1 or 2, in which Signal represents a superparamagnetic nanoparticle coated with an organic layer, preferably having an iron oxide core.

9. Compound according to Claim 8, in which the organic layer is a gem-bisphosphonate layer.

10. Compound according to Claim 1 or 2, in which Signal represents a lipid nanoparticle comprising at least one chelate, advantageously chosen from: DOTA, DTPA, DO3A, HPDO3A, TRITA, TETA, BOPTA, NOTA, PCTA, DOTMA, AAZTA, HOPO and their derivatives.

11. Composition for medical imaging, comprising at least one compound of general formula (I) according to one of Claims 1 to 10 and a pharmaceutically acceptable excipient, advantageously for parenteral administration.

12. Method for preparing the composition according to Claim 11, comprising the addition of a compound of formula (I) according to one of Claims 1 to 10 to an injectable medium comprising the pharmaceutically acceptable excipient.

13. Compound of general formula (I) according to Claims 1 to 10, or compound of formula (I) Signal-Linker-Peptide in which Linker and Peptide are as defined in Claims 1 to 9 and Signal represents a lipid nanoparticle, said nanoparticle bearing at least one chelate capable of being coupled to a paramagnetic metal, for its use as an agent for diagnosing diseases associated with apoptosis.

14. Use of a compound (I) according to one of Claims 1 to 10, or compound of formula (I) Signal-Linker-Peptide in which Linker and Peptide are as defined in Claims 1 to 9 and Signal represents a lipid nanoparticle, said nanoparticle bearing at least one chelate capable of being coupled to a paramagnetic metal, for the preparation of a composition for diagnosing diseases associated with apoptosis, advantageously chosen from chronic neurodegenerative diseases, ischemic diseases and inflammatory pathological conditions.

15. Use of an apoptosis-targeting peptide of formula defined in Claim 1, for the preparation of a composition for diagnosing diseases associated with apoptosis.


### SEQUENCE LISTING

<110> GUERBET PORT, Marc ROUSSEAUX, Olivier MULLER, Robert BURTEA, Carmen
<120> Composés pour le diagnostique de l'apoptose
<130> D25236
<150> FR0754086
   <151> 2007-03-28
<160> 11
<170> PatentIn version 3.3
<210> 1
   <211> 6
   <212> PRT
   <213> Artificial
<220>
   <223> marqueur de l'apoptose
<220>
   <221> VARIANT
   <222> (1)..(1)
   <223> replace="isoleucine"
<220>
   <221> VARIANT
   <222> (2)..(2)
   <223> replace="isoleucine"
<220>
   <221> MISC_FEATURE
   <222> (12) .. ()
   <223> note="Les acides aminés figurant dans la séquence n'ont aucune préférence par rapport aux acides aminés cités dans les annotations ci-dessus pour les dites positions"
<400> 1
   Leu Leu Lys Lys Pro Phe
   1 5
<210> 2
   <211> 6
   <212> PRT
   <213> Artificial
<220>
   <223> marqueur de l'apoptose
<220>
   <221> VARIANT
   <222> (5)..(5)
   <223> replace="leucine"
<220>
   <221> MISC_FEATURE
   <222> (5) .. (5)
   <223> note="Les acides aminés figurant dans la séquence n'ont aucune préférence par rapport aux acides aminés cités dans les annotations ci-dessus pour les dites positions"
<400> 2
   Asp Ala His Ser Phe Ser
   1 5
<210> 3
   <211> 6
   <212> PRT
   <213> Artificial
<220>
   <223> marqueur de l'apoptose
<220>
   <221> VARIANT
   <222> (5)..(5)
   <223> replace="valine"
<220>
   <221> VARIANT
   <222> (6) .. (6)
   <223> replace="arginine"
<220>
   <221> MISC_FEATURE
   <222> (56) .. ()
   <223> note="Les acides aminés figurant dans la séquence n'ont aucune préférence par rapport aux acides aminés cités dans les annotations ci-dessus pour les dites positions"
<400> 3
   Pro Gly Asp Leu Ser Thr
   1               5
<210> 4
   <211> 6
   <212> PRT
   <213> Artificial
<220>
   <223> marqueur de l'apoptose
<220>
   <221> VARIANT
   <222> (3) .. (3)
   <223> replace="histidine"
<220>
   <221> VARIANT
   <222> (6)..(6)
   <223> replace="isoleucine"
<220>
   <221> MISC_FEATURE
   <222> (36) .. ()
   <223> note="Les acides aminés figurant dans la séquence n'ont aucune préférence par rapport aux acides aminés cités dans les annotations ci-dessus pour les dites positions"
<400> 4
   His Gly Asp Leu Ser Thr
   1               5
<210> 5
   <211> 6
   <212> PRT
   <213> Artificial
<220>
   <223> marqueur de l'apoptose
<400> 5
   Val Leu Gly Glu Arg Gly
   1               5
<210> 6
   <211> 6
   <212> PRT
   <213> Artificial
<220>
   <223> marqueur de l'apoptose
<220>
   <221> VARIANT
   <222> (1)..(1)
   <223> replace="isoleucine"
<220>
   <221> VARIANT
   <222> (1)..(1)
   <223> replace="valine"
<220>
   <221> VARIANT
   <222> (1)..(1)
   <223> replace="alanine"
<220>
   <221> VARIANT
   <222> (2) .. (2)
   <223> replace="leucine"
<220>
   <221> VARIANT
   <222> (2)..(2)
   <223> replace="valine"
<220>
   <221> VARIANT
   <222> (2)..(2)
   <223> replace="alanine"
<220>
   <221> VARIANT
   <222> (3)..(3)
   <223> replace="arginine"
<220>
   <221> VARIANT
   <222> (3)..(3)
   <223> replace="histidine"
<220>
   <221> VARIANT
   <222> (3)..(3)
   <223> replace="ornithine"
<220>
   <221> VARIANT
   <222> (4)..(4)
   <223> replace="arginine"
<220>
   <221> VARIANT
   <222> (4) .. (4)
   <223> replace="histidine"
<220>
   <221> VARIANT
   <222> (4) .. (4)
   <223> replace="ornithine"
<220>
   <221> VARIANT
   <222> (6)..(6)
   <223> replace="tryptophane"
<220>
   <221> VARIANT
   <222> (6)..(6)
   <223> replace="tyrosine"
<220>
   <221> VARIANT
   <222> (6)..(6)
   <223> replace="histidine"
<220>
   <221> VARIANT
   <222> (6)..(6)
   <223> replace="biphénylalanine"
<220>
   <221> VARIANT
   <222> (6)..(6)
   <223> replace="naphthylalanine"
<220>
   <221> MISC_FEATURE
   <222> (12346)..(6)
   <223> note="Les acides aminés figurant dans la séquence n'ont aucune préférence par rapport aux acides aminés cités dans les annotations ci-dessus pour les dites positions"
<400> 6
   Leu Ile Lys Lys Pro Phe
   1 5
<210> 7
   <211> 6
   <212> PRT
   <213> Artificial
<220>
   <223> marqueur de l'apoptose
<220>
   <221> VARIANT
   <222> (2)..(2)
   <223> replace="leucine"
<220>
   <221> VARIANT
   <222> (2).. (2)
   <223> replace="isoleucine"
<220>
   <221> VARIANT
   <222> (2)..(2)
   <223> replace="valine"
<220>
   <221> VARIANT
   <222> (3)..(3)
   <223> replace="lysine"
<220>
   <221> VARIANT
   <222> (3)..(3)
   <223> replace="arginine"
<220>
   <221> VARIANT
   <222> (4)..(4)
   <223> replace="thréonine"
<220>
   <221> VARIANT
   <222> (5)..(5)
   <223> replace="leucine"
<220>
   <221> VARIANT
   <222> (5)..(5)
   <223> replace="isoleucine"
<220>
   <221> VARIANT
   <222> (5)..(5)
   <223> replace="valine"
<220>
   <221> VARIANT
   <222> (6)..(6)
   <223> replace="thréonine"
<220>
   <221> MISC_FEATURE
   <222> (23456) .. ()
   <223> note="Les acides aminés figurant dans la séquence n'ont aucune préférence par rapport aux acides aminés cités dans les annotations ci-dessus pour les dites positions"
<400> 7
   Asp Ala His Ser Phe Ser
   1               5
<210> 8
   <211> 6
   <212> PRT
   <213> Artificial
<220>
   <223> marqueur de l'apoptose
<220>
   <221> VARIANT
   <222> (4) .. (4)
   <223> replace="isoleucine"
<220>
   <221> VARIANT
   <222> (4) .. (4)
   <223> replace="valine"
<220>
   <221> VARIANT
   <222> (5)..(5)
   <223> replace="valine"
<220>
   <221> VARIANT
   <222> (6)..(6)
   <223> replace="arginine"
<220>
   <221> VARIANT
   <222> (6) .. (6)
   <223> replace="sérine"
<220>
   <221> VARIANT
   <222> (6)..(6)
   <223> replace="lysine"
<220>
   <221> MISC_FEATURE
   <222> (456) .. ()
   <223> note="Les acides aminés figurant dans la séquence n'ont aucune préférence par rapport aux acides aminés cités dans les annotations ci-dessus pour les dites positions"
<400> 8
   Pro Gly Asp Leu Ser Thr
   1               5
<210> 9
   <211> 6
   <212> PRT
   <213> Artificial
<220>
   <223> marqueur de l'apoptose
<220>
   <221> VARIANT
   <222> (1) .. (1)
   <223> replace="lysine"
<220>
   <221> VARIANT
   <222> (1) .. (1)
   <223> replace="arginine"
<220>
   <221> VARIANT
   <222> (2)..(2)
   <223> replace="glycine"
<220>
   <221> VARIANT
   <222> (3) .. (3)
   <223> replace="histidine"
<220>
   <221> VARIANT
   <222> (3)..(3)
   <223> replace="lysine"
<220>
   <221> VARIANT
   <222> (3)..(3)
   <223> replace="arginine"
<220>
   <221> VARIANT
   <222> (4)..(4)
   <223> replace="valine"
<220>
   <221> VARIANT
   <222> (4) .. (4)
   <223> replace="leucine"
<220>
   <221> VARIANT
   <222> (4) .. (4)
   <223> replace="arginine"
<220>
   <221> VARIANT
   <222> (6) .. (6)
   <223> replace="isoleucine"
<220>
   <221> VARIANT
   <222> (6)..(6)
   <223> replace="sérine"
<220>
   <221> VARIANT
   <222> (6)..(6)
   <223> replace="leucine"
<220>
   <221> VARIANT
   <222> (6)..(6)
   <223> replace="valine"
<220>
   <221> MISC_FEATURE
   <222> (12346)..(6)
   <223> note="Les acides aminés figurant dans la séquence n'ont aucune préférence par rapport aux acides aminés cités dans les annotations ci-dessus pour les dites positions"
<400> 9
   His Asn Asp Ile Ser Thr
   1               5
<210> 10
   <211> 6
   <212> PRT
   <213> Artificial
<220>
   <223> marqueur de l'apoptose
<220>
   <221> VARIANT
   <222> (1) .. (1)
   <223> replace="leucine"
<220>
   <221> VARIANT
   <222> (1) .. (1)
   <223> replace="isoleucine"
<220>
   <221> VARIANT
   <222> (2) .. (2)
   <223> replace="isoleucine"
<220>
   <221> VARIANT
   <222> (2)..(2)
   <223> replace="arginine"
<220>
   <221> VARIANT
   <222> (2)..(2)
   <223> replace="valine"
<220>
   <221> VARIANT
   <222> (4)..(4)
   <223> replace="acide glutamique"
<220>
   <221> VARIANT
   <222> (5) .. (5)
   <223> replace="arginine"
<220>
   <221> MISC_FEATURE
   <222> (1245) .. ()
   <223> note="Les acides aminés figurant dans la séquence n'ont aucune préférence par rapport aux acides aminés cités dans les annotations ci-dessus pour les dites positions"
<400> 10
   Val Leu Gly Asp Lys Gly
   1 5
<210> 11
   <211> 6
   <212> PRT
   <213> Artificial
<220>
   <223> marqueur de l'apoptose
<400> 11
   Leu Ile Lys Lys Pro Phe
   1 5